# EUROPEAN PATENT APPLICATION

(11) **EP 4 454 650 A1**
(43) Date of publication of application: **30.10.2024**
(21) Application number: 22911995.3
(22) Date of filing: 22.12.2022
(51) Int. Cl.: A61K 31/522, A61P 31/12, A61P 37/00, A23L 33/10, A23K 20/137, A61K 31/708, A23L 33/125

(54) **ANTIVIRAL COMPOSITION COMPRISING NUCLEOSIDE ANALOGUES DERIVED FROM NUCLEIC ACID AND PHARMACEUTICALLY ACCEPTABLE SALT THEREOF**

(30) Priority: 22.12.2021 KR 20210185397; 12.08.2022 KR 20220101489
(71) Applicant: CJ CheilJedang Corporation, Seoul 04560 (KR)
(72) Inventor: MOON, Ho Jin, Seoul 04560 (KR); KIM, Young Nam, Seoul 04560 (KR); LEE, Na-Ra, Seoul 04560 (KR); LEE, Changsuk, Seoul 04560 (KR); CHO, Ah Reum, Seoul 04560 (KR)
(74) Representative: Reddie & Grose LLP
(86) International application number: PCT/KR2022/021110
(87) International publication number: WO 2023/121362

(57) **Abstract**

The present application relates to an antiviral composition comprising nucleoside analogues derived from a nucleotide, and a composition, a feed or a feed additive for enhancing immunity.

## Description

### [TECHNICAL FIELD]

The present application relates to an antiviral composition, an immunomodulatory composition, and a feed composition, which comprise nucleoside analogues derived from nucleic acid and a pharmaceutically acceptable salt thereof.

### [BACKGROUND ART]

Damage to people and animals caused by viral causes is gradually increasing worldwide, and there is a trend that the damage scale is also increasing. The significant impact on humanity of recently occurring or ongoing zoonotic viruses such as SARS-CoV-2, MERS, Influenza and the like, and the social and economical damage caused by animal viruses such as avian influenza and African swine fever is further urging preparation of effective measures to control viral diseases.

In particular, there will be no disagreement that complete control of viral diseases is impossible in the current situation where only a few vaccines are the only countermeasures against viruses. As a powerful solution to solve this, efforts to develop an animal virus therapeutic agent have continued for the past several decades. Research on viral therapeutic agents (viral infection and proliferation inhibitors) for controlling various animal viruses such as Porcine Reproductive and Respiratory Syndrome virus (PRRSV), Porcine Epidemic Diarrhea virus (PEDV), Influenza Virus such as Swine Influenza virus (SIV) or Avian Influenza virus (AIV), Porcine Rotavirus (PRV), Porcine Circovirus (PCV), Avian Infectious Bronchitis virus (IBV), Avian Newcastle Disease virus (NDV), Bovine Viral Diarrhea virus (BVDV), Bovine Rotavirus (BRV) and Canine Parvovirus (CPV) is continuing (Kim et al., 2013 Virus Res. 171(1):44-53, Paul C Jordan et al., 2018 Antivir Chem Chemother. 26:1-19, Andrea J Pruijssers et al., 2019 Current Opinion in Virology 35:57-62), but highly effective antivirals have not yet been commercialized, and now that viral diseases are threatening as a great threat, it can be said that needs for development and commercialization of antivirals are very high.

### [DISCLOSURE]

### [TECHNICAL PROBLEM]

An object of the present application is to provide an antiviral composition, an immunomodulatory composition, and a feed composition, which comprise nucleoside analogues derived from nucleic acid and a pharmaceutically acceptable salt thereof.

### [TECHNICAL SOLUTION]

One of one aspect of the present application may be an antiviral composition comprising nucleosides in a dialdehyde form of inosine, a dialdehyde form of xanthosine, a dialdehyde form of guanosine, or an acyclic diol form of inosine, an acyclic diol form of xanthosine, an acyclic diol form of guanosine.

One of one aspect of the present application may be an immunomodulatory composition comprising nucleosides in a dialdehyde form of inosine, a dialdehyde form of xanthosine, a dialdehyde form of guanosine, or an acyclic diol form of inosine, an acyclic diol form of xanthosine, an acyclic diol form of guanosine.

One of one aspect of the present application may be a medicine, feed or feed additive.

Specifically, the composition may be a composition showing an antiviral effect against at least one virus selected from the group consisting of Porcine Reproductive and Respiratory Syndrome virus (PRRSV), Porcine Epidemic Diarrhea virus (PEDV), Influenza Virus such as Swine Influenza virus (SIV) or Avian Influenza virus (AIV), Porcine Rotavirus (PRV), Porcine Circovirus (PCV), Avian Infectious Bronchitis virus (IBV), Avian Newcastle Disease virus (NDV), Bovine Viral Diarrhea virus (BVDV), Bovine Rotavirus (BRV) and Canine Parvovirus (CPV).

### [ADVANTAGEOUS EFFECTS]

The nucleoside analogues derived from nucleic acid can be usefully used as an antiviral agent.

### [BRIEF DESCRIPTION OF DRAWINGS]

FIG. 1a is a diagram showing that IMP, XMP and GMP are converted into a dialdehyde form to obtain the structures of nucleosides in a dialdehyde form of Chemical formulas 1 to 3 according to the present application.
FIG. 1b shows the chemical structures of the nucleosides of Chemical formulas 4 to 6 according to the present application, obtained by converting IMP, XMP and GMP are converted into an acyclic diol form, respectively.
FIG. 2 is a graph showing the anti-PRRSV efficacy shown by inosine in a dialdehyde form confirmed through immunofluorescence staining method (top) and the PRRSV infectivity using this (bottom).
FIG. 3 is a graph showing the anti-PRRSV efficacy shown by xanthosine in a dialdehyde form confirmed through immunofluorescence staining method (top) and the PRRSV infectivity using this (bottom).
FIG. 4 is a graph showing the anti-PRRSV efficacy shown by guanosine in a dialdehyde form confirmed through immunofluorescence staining method (top) and the PRRSV infectivity using this (bottom).
FIG. 5 is a graph showing the anti-PRRSV efficacy shown by inosine in an acyclic diol form confirmed through immunofluorescence staining method (top) and the PRRSV infectivity using this (bottom).
FIG. 6 is a graph showing the anti-PRRSV efficacy shown by xanthosine in an acyclic diol form confirmed through immunofluorescence staining method (top) and the PRRSV infectivity using this (bottom).
FIG. 7 is a graph showing the anti-PRRSV efficacy shown by guanosine in an acyclic diol form confirmed through immunofluorescence staining method (top) and the PRRSV infectivity using this (bottom).
FIG. 8 is the result showing the concentration of the material in the blood over time after inoculating xanthosine in a dialdehyde form in Oral (Intragastric) and IP (Intraperitoneal) methods.
FIG. 9 is the result showing the concentration of the material in the blood over time after inoculating guanosine in a dialdehyde form in Oral (Intragastric) and IP (Intraperitoneal) methods.
FIG. 10 shows a test schedule for an influenza virus challenge test using mice.
FIG. 11 shows the change in body weight of mice of each group during the 7-day monitoring period after SIV infection.
FIG. 12 shows the change in survival rate of mice of each group during the 7-day monitoring period after SIV infection.
FIG. 13 shows the result of observing lung tissue lesions extracted through autopsy on the 3rd, 5th and 7th days after SIV infection by each group.
FIG. 14 shows the result of measuring virus titers remaining in lung tissue extracted through autopsy on the 3rd, 5th and 7th days after SIV infection by PCR method by each group.
FIG. 15 is representative photographs of lung tissue lesions for each group at the final autopsy on the 7th day after SIV infection.
FIG. 16 shows the test schedule of the PRRS virus challenge test using piglets.
FIG. 17 shows the change in body weight of piglets in each group during the 14-day monitoring period after PRRSV infection.
FIG. 18 is the result of comparing the amount of viruses present in lung tissue collected from piglets in each group at the 2nd week after PRRSV infection with positive controls.
FIG. 19 is photographs of visually observing interstitial pneumonia lesions by extracting lungs of representative piglets of each group at the 2nd week after PRRSV infection.
FIG. 20 is photographs of observing PRRS virus particles present in tissue by immunohistochemistry (IHC) by extracting lungs of representative piglets of each group at the 2nd week after PRRSV infection.

### [BEST MODE]

Hereinafter, the present application will be described in detail.

The antiviral or immunomodulatory composition of the present application is characterized by comprising nucleoside analogs in the dialdehyde form and acyclic diol form, as one specific aspect, which are at least one selected from the group consisting of nucleic acid-derived inosine, xanthosine and guanosine in the dialdehyde form and acyclic diol form represented by the following Chemical formulas 1 to 6, and pharmaceutically acceptable salts thereof as an active ingredient.

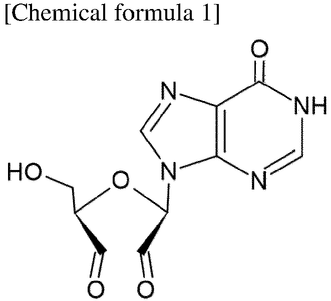

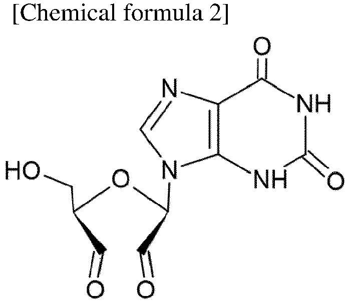

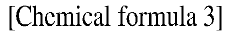

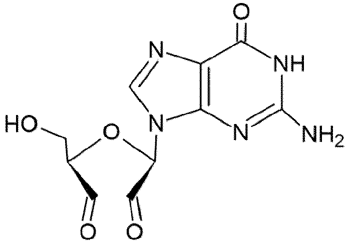

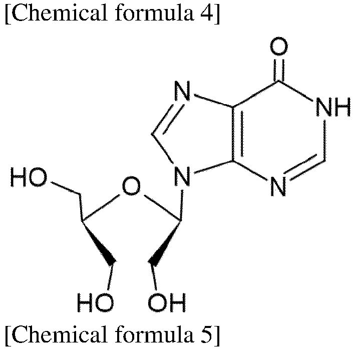

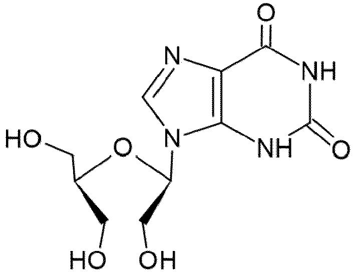

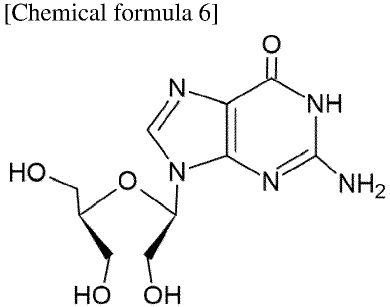

This active ingredient, inosine, xanthosine or guanosine in the dialdehyde form or acyclic diol form may be produced through an optimized process. As one specific embodiment, it may be prepared by the method described in examples according to the present invention, but not limited thereto.

Then, based on 100 parts by weight of the antiviral composition, the compound represented by the Chemical formulas 1 to 6 or pharmaceutically acceptable salts thereof may be contained by 0.0001 part by weight to 20 parts by weight, 0.0001 part by weight to 15 parts by weight, 0.0001 part by weight to 10 parts by weight, 0.0001 to 5 parts by weight, 0.0001 part by weight to 1 part by weight, 0.0001 to 0.5 parts by weight, 0.0001 to 0.1 part by weight, 0.0001 to 0.05 parts by weight, 0.0001 to 0.01 part by weight, 0.0001 to 0.001 part by weight, 0.0001 to 0.0005 parts by weight, 0.001 part by weight to 20 parts by weight, 0.001 part by weight to 15 parts by weight, 0.001 part by weight to 10 parts by weight, 0.001 to 5 parts by weight, 0.001 part by weight to 1 part by weight, 0.001 to 0.5 parts by weight, 0.001 to 0.1 part by weight, 0.001 to 0.05 parts by weight, 0.001 to 0.01 part by weight, 0.001 to 0.005 parts by weight, 0.01 part by weight to 20 parts by weight, 0.01 part by weight to 15 parts by weight, 0.01 part by weight to 10 parts by weight, 0.01 to 5 parts by weight, 0.01 part by weight to 1 part by weight, 0.01 to 0.5 parts by weight, 0.01 to 0.1 part by weight, 0.01 to 0.05 parts by weight, 0.1 part by weight to 20 parts by weight, 0.1 part by weight to 15 parts by weight, 0.1 part by weight to 10 parts by weight, 0.1 to 5 parts by weight, 0.1 part by weight to 1 part by weight, 0.1 to 0.5 parts by weight, 1 to 20 parts by weight, 1 to 15 parts by weight, 1 to 10 parts by weight, 1 to 5 parts by weight, 5 to 20 parts by weight, 5 to 15 parts by weight, 5 to 10 parts by weight, 10 to 15 parts by weight, 10 to 20 parts by weight, or 15 to 20 parts by weight, and therefore, it is not preferable that when the compound or pharmaceutically acceptable salts thereof are comprised in less than 0.0001 part by weight, the antiviral effect of the compound is not properly exhibited, and when it is comprised more than 20 parts by weight, the amount of increase of the antiviral effect is insignificant compared to the amount of increase in compound content.

The virus is not limited thereto, as long as it is any type of virus, but may be at least one selected from the group consisting of Porcine Reproductive and Respiratory Syndrome virus (PRRSV), Porcine Epidemic Diarrhea virus (PEDV), Influenza Virus such as Swine Influenza virus (SIV) or Avian Influenza virus (AIV), Porcine Rotavirus (PRV), Porcine Circovirus (PCV), Avian Infectious Bronchitis virus (IBV), Avian Newcastle Disease virus (NDV), Bovine Viral Diarrhea virus (BVDV), Bovine Rotavirus (BRV) and Canine Parvovirus (CPV).

In the present application, description for representative viruses among viruses of which antiviral efficacy is verified is as follows.

Porcine reproductive and respiratory syndrome virus (PRRSV) is a representative chronic wasting disease in pigs, and belongs to Ateriviridae, and is the order of Nidovirales, and is a distant relative of Coronaviridae. This virus is largely divided into European type (EU strain) and North American type (NA strain) based on antigen and genetic characteristics, and has an envelope protein and has positive single strand RNA of 15 kb as a gene. There are at least 10 or more open reading frames (ORFs) in this gene, and ORFla and ORFlb at the 5' end encoding a nonstructural protein (NSP) account for two-thirds of the entire gene. This virus is known as a virus causing very serious damage, as it has characteristics to be infected into macrophages (porcine alveolar macrophage (PAM)) mainly present in lungs and therefore, it makes them vulnerable to secondary infection from other viruses or bacteria. Infected pregnant sows may experience miscarriage and stillbirth (mummification), and in piglets, severe respiratory symptoms and mortality may occur. Along with Porcine Circovirus, damage of post-weaning multisystemic wasting syndrome has long been a big problem in the pig farming industry.

Porcine Epidemic Diarrhea virus (PEDV) belonging to the Coronavirus family, is a virus which infects small intestine of pigs as its name itself and eliminating villi and causes severe dehydration and diarrhea, and causes serious economic losses to farms, and in particular, it is a viral disease which causes greater economic losses, as it shows a high mortality rate (50-100%) in young pigs. PEDV has an envelope, and has positive single strand RNA of about 28 kb, and contains 7 ORFs. It is largely divided into two genetic types (G1, G2) by Spike gene, and each type is divided into a, b again. Recently, G2c type has been also discovered. Additionally, S INDEL (G1b type) which contains many deletions and insertions in S1 subunit of Spike protein and non-S INDEL (G2b type) that does not are present, and the G2b type generally show high viral virulence. Since cross-immunity between G1 and G2 types is not formed well, vaccine development must be developed suitably for each type. It is a situation that there is still a lack of definite countermeasures against PEDV, as there is no therapeutic agent and vaccines do not form cross-immunity for each type and the like.

Porcine circovirus first identified in 1982 is a causative agent of postweaning multisystemic wasting syndrome (PMWS), and occurs in North America, England, and various countries of Europe, and in Korea, its occurrence began to be confirmed in 1999 and it causes viral diseases in pig farms. Porcine circovirus, the smallest form of animal viruses, is divided into serotypes 1 and 2, and it is known that the type 1 is non-pathogenic, whereas the type 2 is a pathogenic virus. In addition, many cases of mixed infection with Porcine reproductive and respiratory syndrome (PRRS) virus or Porcine parvovirus have been reported in farms, and it is a troublesome virus that causes serious economic problems.

IBV belonging to coronavirus has been occurring worldwide, and domestic outbreak was officially confirmed in 1986. It has been reported that there are viruses isolated all over the world such as Massachusetts type, whereas there are also viruses showing regional characteristics prevalent in Europe, America, Southeast Asia and the like, respectively. In Korea, a virus causing egg laying reduction and respiratory symptoms was first isolated in 1986, and since 1990, virus infection with kidney types as main symptoms has been occurring at a high frequency continuously. Recently prevalent isolated strains are largely divided into respiratory type Korean I group and kidney type Korean II group, and in case of the kidney type, it is analyzed to be similar to Japanese or Chinese isolated strains.

BVDV causes bovine viral diarrhea, a national notifiable infectious disease type 2 in cattle, and causes ulcers and diarrhea in digestive tract mucous membranes, respiratory lesions and the like, and in severe cases, even mortality of cattle is caused. In 1946, it was first reported by Olafson et al. in the United States. BVDV is an RNA virus classified into the pestivirus genus of Flaviviridae, and depending on whether it causes cytopathic effects in cell lines, it is classified into cytopathic strains (NADL, Singer, T20 strain, etc.) and no-cytopathic strains (New Work strain, etc.). Main routes are oral infection and placental infection due to feed contaminated with feces as the infection route of BVDV, but respiratory and reproductive infections are also possible. The reproductive infection is known to be transmitted through semen and fertilized eggs.

CPV infectious disease occurred and was reported worldwide in 1978, and in Korea, it was first confirmed in Gyeonggi-do in the 1980s, and after that, it occurred nationwide. It may be largely divided clinically or pathologically into two types, which are a cardiac type that can be seen mainly in 3-8-week-old young dogs and an enteritis type that can be seen in postweaning dogs. Symptoms of the enteritis type include severe vomiting, dehydration due to bloody diarrhea, reduction in white blood cells, and death. There is no therapeutic agent against CPV, and there are intravenous fluid injections for alleviating symptoms and antibiotic administration for inhibiting complex bacterial infection. For a preventive purpose, inoculation of an attenuated vaccine and an inactivated vaccine is performed as the only means.

Porcine Rotavirus infectious disease is one of major viral diseases that cause diarrhea and inhibit growth performance in young pigs along with Transmissible gastroenteritis virus (TGE) and epidemic diarrhea (PED, Porcine epidemic diarrhea). The mortality rate is around 10%, but in case of mixed infection, the mortality rate increases. It is prevented with a vaccine, but complete control through the vaccine is not easy, since it is an RNA virus in which mutations occurs easily and there are a total of 8 serotype groups from A to H depending on VP6 capsid protein (among them, type A causes greatest damage). Furthermore, since recombinant rotaviruses that cause not only intra-species rotavirus infection, but also interspecies infection may be easily created, measures other than vaccines should be quickly prepared for rotavirus infectious diseases which may cause significant damage due to a new recombinant rotavirus.

In addition to pigs, in calves, particularly, young calves 2-4 weeks old, severe diarrhea is caused due to infection of rotavirus (Bovine Rotavirus (BRV)), and due to this, severe economic damage is being caused. It is known as a cause of disease causing diarrhea in young calves with bovine coronavirus (BCV), bovine viral diarrhea virus (BVDV), and the like, which cause bovine diarrhea worldwide, and in Korea, among them, rotavirus infection is the most serious. As same as in pigs, a preventive effect is expected with a vaccine, but there is no treatment method, so it is difficult to completely control diseases. The bovine rotavirus is structurally divided into 11 types of P type viruses classified by VP4 protein, and 12 types of G type viruses classified by VP7 protein, which constitute the outermost layer of rotavirus.

Newcastle disease is a disease occurring in birds by Newcastle Disease Virus, and 12 genotypes (Paramyxovirus (PMV)-1 ~ PMV12) are present, and PMV-1 is the most important disease cause in birds, particularly, chickens and turkeys and the like. In addition, in chickens, it is classified by pathogenicity into a lentogenic strain with weak pathogenicity, a mesogenic strain with pathogenicity at a moderate level, and a velogenic strain with strong pathogenicity, and the lentogenic strains are used as live-attenuated virus vaccines. It is a notifiable type 1 livestock infectious disease (Korea), which causes 100% mortality within 2~14 days in unvaccinated chickens, and representative Newcastle disease symptoms may include torticollis (symptom of a twisted and turned neck) as a neurological symptom, in addition to difficulty in breathing and cough, green feces or bloody feces. In addition to a high mortality rate, reduction of growth performance and reduction of an egg laying rate may cause enormous damage to farms, and prevention is effective, but due to the nature of RNA viruses, mutant strains can easily occur, so effective treatment methods or substances should be developed to achieve complete disease control.

Swine Influenza virus infects pigs and causes a highly contagious acute respiratory disease. As symptoms, upper respiratory symptoms such as fever, cough, tears, weakness, loss of appetite, and the like are shown, and it inhibits growth performance of pigs to cause damage to farms, although the mortality rate is not very high at 1-4%. Thus, there is a recent trend of vaccination also in pigs, but it is a virus in which mutations occur frequently, and it is difficult to expect complete prevention efficacy, and control combined with a therapeutic agent is necessary. As a zoonotic infectious disease, in 2009, swine influenza A virus H1N1 infected humans, thereby occurring swine flu internationally widely. In general, there are 4 types such as viruses A, B, C, D and the like in the influenza virus, and in pigs, viruses belonging to type A are the main cause of disease. There are H1~H18 and N1~N11 depending on the structure (sequence) of hemagglutinin and neuraminidase proteins that are present in the outer membrane of the virus and play an important role in penetration and budding out in infected cells, and there may be a total of 198 or more subtypes of the virus depending on the combination of these two protein types.

In addition to swine influenza virus diseases, the influenza virus in birds is classified into a highly pathogenic (H5N1, H5N6, H5N8, H7N9, etc.) virus with a mortality rate of up to 100%, and a low pathogenic (H9N2, etc.) which has a low mortality rate, but causes great damage to farms by reducing productive performance (growth and egg laying rate). As it is an RNA virus as same as in pigs, mutations are frequent and occurrence of recombinant viruses occurs frequently, and therefore, it is difficult to expect effects of a vaccine and it is not known which enormous damage may be caused by the recombinant viruses (it may be a huge disaster even for humans as a zoonotic infectious disease), so measures other than vaccines (securing a therapeutic agent) should be prepared to control the disease.

As the pharmaceutically acceptable salt, an acid addition salt formed by a pharmaceutically acceptable free acid is useful. As the free acid, inorganic acid and organic acid may be used, and as the inorganic acid, hydrochloric acid, bromic acid, sulfuric acid, sulfurous acid, phosphoric acid, and the like may be used, and as the organic acid, citric acid, acetic acid, maleic acid, fumaric acid, glucosan, methane sulfonate, acetic acid, glycolate, succinic acid, tartaric acid, 4-toluene sulfonate, galacturonic acid, embonic acid, glutamic acid, citric acid, aspartic acid, and the like may be used. In addition, the pharmaceutical composition containing the nucleoside analogues represented by Chemical formulas 1 to 6 of the present application may comprise not only the pharmaceutically acceptable salts, but also all salts, hydrates and solvates which can be prepared by a common method.

The addition salt according to the present application may be prepared by a common method, and for example, it may be prepared by dissolving a compound selected from the group consisting of compounds represented by Chemical formulas 1 to 6 in a water miscible organic solvent, for example, acetone, methanol, ethanol, or acetonitrile, or the like, and adding an excessive amount of organic acid or adding an acid aqueous solution of inorganic acid, and then immersing or crystallizing. Subsequently, it may be prepared by evaporating the solvent or the excessive amount of acid in this mixture and drying it to obtain an addition salt or suction filtering a precipitated salt.

In addition, the composition may be selected from a pharmaceutical composition or a health food composition.

In one specific example, when the antiviral composition is a pharmaceutical composition, it may further comprise at least one additive selected from the group consisting of carriers, excipients, disintegrating agents, sweeteners, covering agents, swelling agents, lubricants, glidants, flavoring agents, antioxidants, buffer, bacteriostatic agents, diluents, dispersants, surfactants, binders and lubricants, commonly used for preparation of the pharmaceutical composition. Specifically, as the carrier, excipient and diluent, lactose, dextrose, sucrose, sorbitol, mannitol, xylitol, erythritol, maltitol, starch, acacia gum, alginate, gelatin, phosphate calcium, calcium silicate, cellulose, methyl cellulose, microcrystalline cellulose, polyvinyl pyrrolidone, water, methyl hydroxy benzoate, propyl hydroxy benzoate, talc, magnesium stearate and mineral oils may be used, and solid preparations for oral administration include tablets, pills, powders, granules, capsules and the like, and such solid preparations may be prepared by mixing at least one excipient, for example, starch, calcium carbonate, sucrose or lactose, gelatin, and the like to the composition. Furthermore, in addition to simple excipients, lubricants such as magnesium stearate and talc may be used. Oral liquid preparations include suspensions, oral liquids, emulsions, syrup, and the like, and in addition to water and liquid paraffin which are simple diluents commonly used, various excipients, for example, wetting agents, sweeteners, flavoring agents, preservatives and the like may be comprised. Preparations for parenteral administration include sterilized aqueous solutions, non-aqueous solvents, suspensions, emulsions, freeze-drying agents, suppositories and the like may be used. As the non-aqueous solvents or suspensions, propylene glycol, polyethylene glycol, vegetable oils such as olive oil, injectable esters such as ethyl oleate and the like may be used.

As other embodiment, the pharmaceutical composition may be used as formulated as granules, powders, coated tablets, tablets, pills, capsules, suppositories, gel, syrup, suspensions, emulsions, instillates, or liquid. According to one example of the present application, the pharmaceutical composition may be administered into a subject by a method well-known in the art, for example, by a common method through an oral, intravenous, intra-arterial, intraperitoneal, intramuscular, subcutaneous, intraperitoneal, intrasternal, dermal, intranasal, inhalation, local, intra-rectal, oral, intraocular, or intradermal route, but not limited thereto.

A specific dose of the compound represented by Chemical formulas 1 to 6 or pharmaceutically acceptable salt thereof may differ depending on the subject's condition and body weight, kind and degree of disease, drug form, administration route and period, and it may be selected appropriately by those skilled in the art.

According to one example of the present application, it is not limited thereto, but as specified below, in case of intragastric administration of an appropriate amount for each livestock species into animals, it may be absorbed through intestinal epithelial cells and enter the bloodstream and spreads to each organ tissue, and in particular, be delivered/absorbed to cells present in tissue in which viruses proliferate and inhibit virus proliferation in the cells infected by virus challenge, and finally, it may be used as a therapeutic agent of an animal infected by a virus.
- Dogs under 6 months old: 100mg/kg, dogs over 6 months old: 200mg/kg, administration twice a day, daily administration for 4 weeks or more
- Cats under 6 months old: 100mg/kg, cats over 6 months old: 200mg/kg, administration twice a day, daily administration for 4 weeks or more
- Chickens under 1 week old: 20mg/kg, chickens under 2 weeks old: 50mg/kg, chickens over 3 weeks old: 100mg/kg, administration twice a day or administration mixed according to daily feed intake, daily administration for 4 weeks or more
- Pigs under 4 weeks old: 100mg/kg, pigs under 8 weeks old: 200mg/kg, pigs over 8 weeks old: 300mg/kg, pigs over 50kg: 400mg/kg, administration twice a day or administration mixed according to daily feed intake, daily administration for 4 weeks or more
- Calves under 2 months old: 200mg/kg, cows under 6 months old: 300mg/kg, cows over 6 months: 400mg/kg, administration twice a day or administration mixed according to daily feed intake, daily administration for 4 weeks or more

However, this is only one example, but the scope of the present application is not limited thereby.

In the present application, the subject may be a mammal, a bird, fish, or an arthropod, but not limited thereto. The mammal subject may be a pig, cow, dog, cat or chicken. The fish subject may be flatfish, salmon, sea bream, eel, rockfish, or trout. The arthropod subject may be a shrimp or lobster.

In other embodiment of the present application, the health food may comprise a compound selected from the group consisting of compounds of Chemical formulas 1 to 6 and pharmaceutically acceptable salts thereof by 0.01 to 90 parts by weight, 0.01 to 50 parts by weight, 0.01 to 10 parts by weight, 0.01 to 5 parts by weight, 0.01 to 1 part by weight, 0.01 to 0.1 part by weight, 0.1 to 90 parts by weight, 0.1 to 50 parts by weight, 0.1 to 10 parts by weight, 0.1 to 5 parts by weight, 0.1 to 1 part by weight, 1 to 90 parts by weight, 1 to 50 parts by weight, 1 to 10 parts by weight, 1 to 5 parts by weight, 10 to 90 parts by weight, 10 to 70 parts by weight, 10 to 50 parts by weight, 10 to 30 parts by weight, or 10 to 20 parts by weight, based on the total health food of 100 parts by weight, but not limited thereto. In other embodiment of the present application, the health food may further comprise at least one additive selected from the group consisting of organic acid, phosphate, antioxidants, lactose casein, dextrin, glucose, sugar and sorbitol. The organic acid is not limited thereto, but may be citric acid, malic acid, adipic acid, lactose or malic acid, and the phosphate is not limited thereto, but may be sodium phosphate, potassium phosphate, acidic pyrophosphate or polyphosphate (polymerized phosphate), and the antioxidant is not limited thereto, but may be a natural antioxidant such as polyphenol, catechin, alpha-tocopherol, rosemary extract, licorice extract, chitosan, tannic acid or phytic acid or the like. In other embodiment of the present application, the health food may contain various nutritional supplements, probiotics, vitamins, minerals (electrolytes), flavoring agents such as synthetic flavoring agents and natural flavoring agents, coloring agents and fillers (cheese, chocolate, etc.), pectic acid and salts thereof, alginic acid and salts thereof, organic acids, protective colloidal thickeners, pH adjusting agents, stabilizers, preservatives, glycerin, alcohol, carbonating agents used for carbonated drinks, and the like. According to one example of the present application, the formulation of the health food is not limited thereto, but may be a solid, powder, granule, tablet, capsule, liquid or beverage form. In addition, the health food is not limited thereto, but may be used for preparation of food such as confectionery, sugars, ice cream products, dairy products, meat products, fish meat products, Tofu or jelly, edible oils and fats, noodles, tea, beverages, special nutritional foods, health supplements, seasoning foods, ice, ginseng products, kimchi pickled foods, dried fish and shells, fruits, vegetables, dried products and cut products of fruits or vegetables, fruit juices, vegetable juices, mixed juices thereof, chips, noodles, processed stock raising foods, processed marine foods, processed dairy products, fermented oil foods, legume foods, grain foods, fermented microbial foods, confectionery and bakery, spicery, processed meat, acidic beverages, licorice, herbs, and the like.

In addition, the present application provides a feed additive containing a compound selected from the group consisting of compounds of Chemical formulas 1 to 6 and pharmaceutically acceptable salts thereof as an active ingredient.

In one example, the feed additive of the present application may be applied in various forms as same as the antiviral composition. Examples of the preparation forms, are not limited thereto, but include liquids, suspensions, powders, granules, tablets, capsules, pills and the like. In addition, for preparation into the forms, at least one kind of additives, excipients, for example, diluents, glidants, binders, disintegrating agents, sweeteners, stabilizers and preservatives which can be comprised in a common feed additive in addition to the active ingredient may be selected and used, and a flavoring, an adjuvant and the like for providing additional functions may be mixed and used. Specifically, the diluent may be lactose, corn starch, soybean oil, microcrystalline cellulose, or mannitol, and the glidant may be magnesium stearate or talc, and the binder may be polyvinyl pyrrolidone or hydroxypropyl cellulose. In addition, the disintegrating agent may be calcium carboxymethyl cellulose, sodium starch glycolate, polacrilin potassium, or crospovidone, and the sweetener may be white sugar, fructose, sorbitol, aspartame, or nucleic acid (IMP, GMP), and the stabilizer may be sodium carboxymethyl cellulose, betacyclodextrin, white beeswax, or xanthan gum, and the preservative may be methyl parahydroxybenzoate, propyl parahydroxybenzoate, or potassium sorbate.

The feed additive may be selected from the group consisting of mammals, fish, birds or arthropods, specifically, pig, cows, chickens, goats, sheep, horses, fish, shrimps, insects, dogs and cats. Specifically, it may be pigs, cows, chickens, dogs or cats, but not limited thereto. The feed additive can inhibit activity of a virus selected from the group consisting of Porcine Reproductive and Respiratory Syndrome virus (PRRSV), Porcine Epidemic Diarrhea virus (PEDV), Influenza Virus such as Swine Influenza virus (SIV) or Avian Influenza virus (AIV), Porcine Rotavirus (PRV), Porcine Circovirus (PCV), Avian Infectious Bronchitis virus (IBV), Avian Newcastle Disease virus (NDV), Bovine Viral Diarrhea virus (BVDV), Bovine Rotavirus (BRV) and Canine Parvovirus (CPV).

The feed additive may be fed to a subject such as a mammal, fish, bird or arthropod, specifically, a pig, cow, chicken, goat, sheep, horse, fish, shrimp, insect, dog or cat, by the same regime and dosage as the antiviral composition according to the present application. In addition, the feeding method may be a feeding method well known commonly in the art, for example, orally feeding by mixing with a feed and the like, but not limited thereto.

In one example, the feed additive of the present application may be added at various ratios, for example, such as 0.01 to 300g, 1g to 200g or 10g to 100g (namely, 0.001 % by weight to 30 % by weight, 0.1 % by weight to 20 % by weight or 1 % by weight to 10 % by weight, based on the total feed dry weight) and the like to a feed of 1kg based on the dry weight, in accordance with the above regime and dosage, but not limited thereto.

The antiviral material selected from the compounds of Chemical formulas 1 to 6, and pharmaceutically acceptable salts thereof comprised in the antiviral composition, pharmaceutical composition or feed additive according to the present application shows a mechanism effectively inhibiting viral infection and proliferation by the method of lowering infectivity by inhibiting smooth viral gene replication by reducing sources of viral gene replication through a mechanism of inhibiting functions of inosine monophosphate dehydrogenase (IMPDH) which produces GMP required when a virus invades cells and conducts gene replication, or causing abnormalities in functional proteins of a virus produced as a final product by being inserted during a replication process as a guanosine analogue in a viral gene replication process.

Mechanisms known for several nucleoside analogues, for example, well-known efficacy mechanisms such as an immunomodulatory effect, an increase in expression of interferon stimulating factors, inhibition of mechanisms of RNA polymerase of a virus, and the like, may also be mechanisms selected from the compounds of Chemical formulas 1 to 6, and pharmaceutically acceptable salts thereof comprised in the antiviral composition, pharmaceutical composition or feed additive according to the present application.

In one example, as a result of evaluating the antiviral efficacy through in vitro cell evaluation method in order to confirm the effects of the nucleosides of Chemical formulas 1 to 6 according to the present application for inhibiting infection or proliferation of various viruses for each livestock species, it was confirmed that an excellent antiviral effect was shown for all the viruses for each livestock species specified above. Furthermore, as a result of confirming the result of evaluating the antiviral effect in vivo by performing intragastric administration of the nucleosides into mice for 5 days after challenge of Influenza virus (2009 Pandemic strain, CA04), it was confirmed that an excellent antiviral effect was shown. In addition, in order to confirm the antiviral efficacy in target animals, it was confirmed that an excellent antiviral effect was shown, when the effect of alleviating viral diseases and the efficacy of reducing viruses in lung tissue were evaluated, by conducting intragastric administration of the nucleosides to piglets for 14 days after challenge of PRRSV (NA strain).

Hereinafter, in order to help understanding of the present application, it will be described in detail by examples. However, the following examples illustrate the contents of the present application only, but the scope of the present application is not limited by the following examples. The examples of the present application are provided to describe the present application more completely to those skilled in the art.

### [MODE FOR INVENTION]

### [Example]

### Example 1: Preparation method of nucleoside analogues using IMP, XMP, GMP

### 1-1. Preparation method of nucleoside in a dialdehyde form

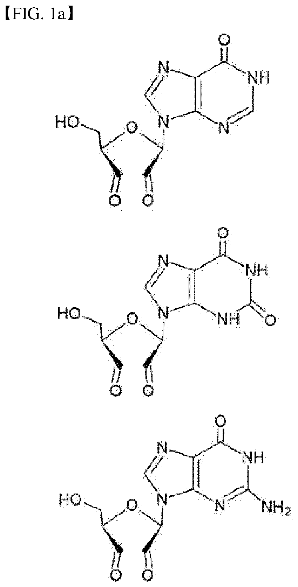

For oxidative cleavage, IMP, XMP, or GMP dissolved in water (prepared and supplied from CJ CheilJedang) was prepared using periodate (NaIO₄) as a catalyst and using an organic solvent, and then was filtered using filter paper and permeates were purified with anion exchange resin (WA30) and then freeze-dried to obtain final products (Maria Meurillon et al., 2014 Eur. J. Med. Chem. 77:18-37). FIG. 1a shows chemical structures of produced inosine, xanthosine and guanosine in a dialdehyde form, which are nucleoside analogues.

### 1-2. Preparation method of nucleoside in an acyclic diol form

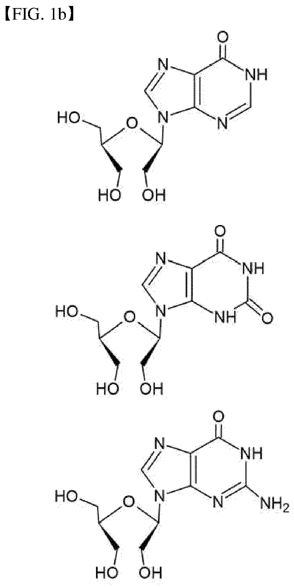

For oxidative cleavage, IMP, XMP, or GMP dissolved in water (prepared and supplied from CJ CheilJedang) was prepared using periodate (NaIO₄) as a catalyst and using an organic solvent, and then was filtered using filter paper and permeates were reduced by adding sodium borohydride (NaBH₄), and then freeze-dried to obtain final products (Maria Meurillon et al., 2014 Eur. J. Med. Chem. 77:18-37). FIG. 1b shows chemical structures of produced inosine, xanthosine and guanosine in an acyclic diol form, which are nucleoside analogues.

### Example 2: Verification of PRRS virus infection efficacy and safety based on in vitro cell evaluation

To evaluate the antiviral and proliferation-inhibiting efficacy against the PRRS virus of the dialdehyde forms and acyclic diol forms of inosine, xanthosine, and guanosine, monkey kidney cells (Marc-145 cell line) were seeded in a 96-well plate at 2×10⁴ cells/well and cultured for one day. The next day, 100 TCID₅₀/ml of PRRSV (NA type, VR-2332 strain) and serially diluted aldehyde and acyclic diol forms of inosine, xanthosine, and guanosine were simultaneously added to the cells. Three days after treatment, the cells were fixed with a 1:1 mixed solution of MeOH/Aceton and sequentially treated with primary antibody using SDOW-17, a PRRSV N (Nucleocaspid) protein binding antibody, and secondary antibody that attaches to the primary antibody and emits fluorescence. Then, cells showing fluorescence were measured to analyze whether viral infection was inhibited. In addition, DAPI (4', 6-diamidino-2-phenylindole) was used to count the number of cells, and the number of infected cells was calculated from the total number of cells, and the efficacy of the material was compared at the quantified infection level.

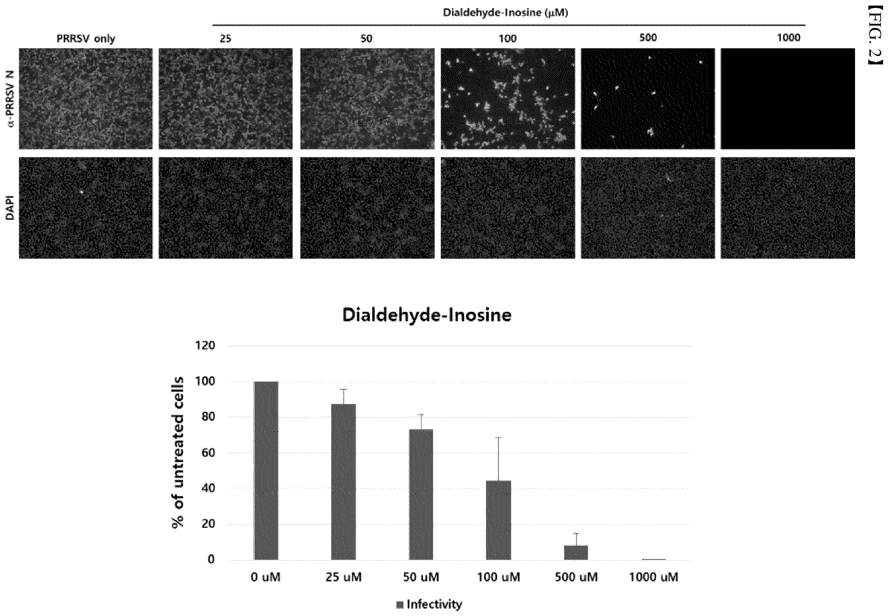

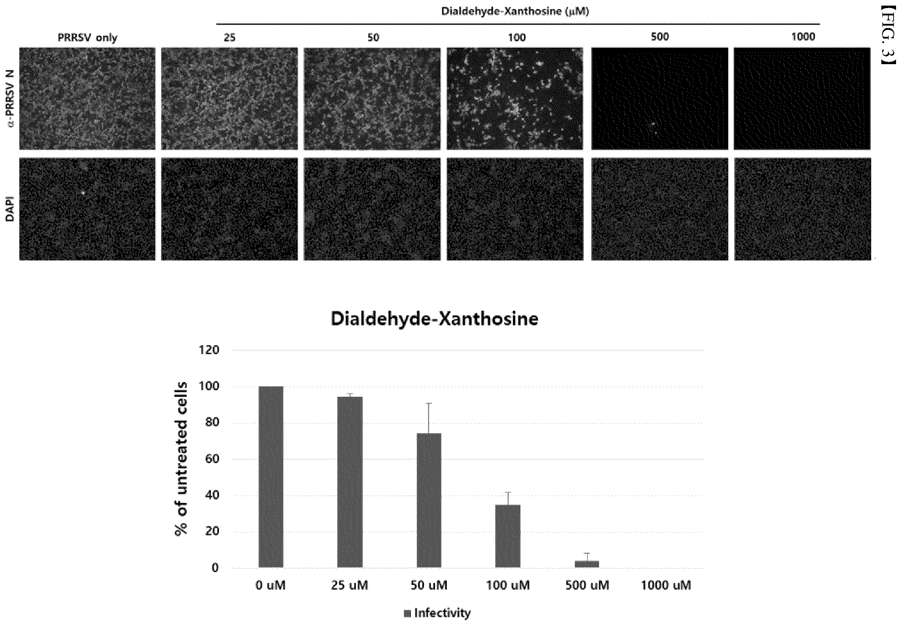

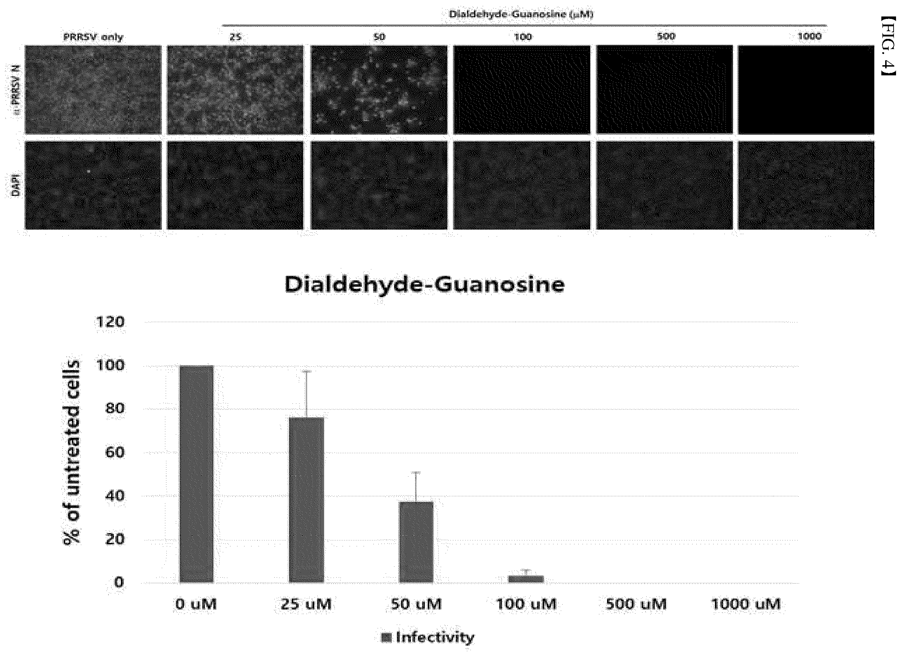

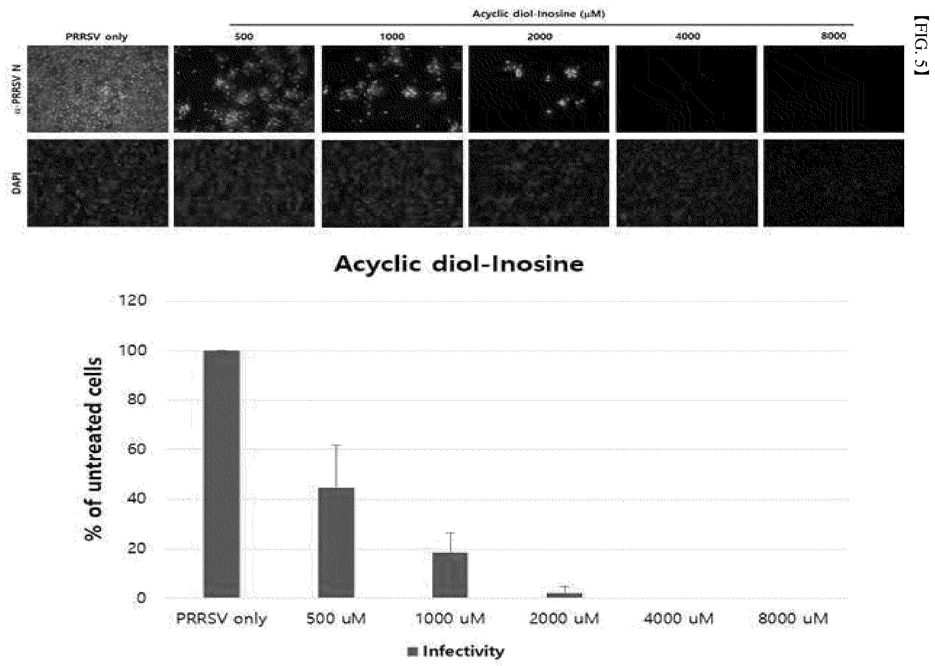

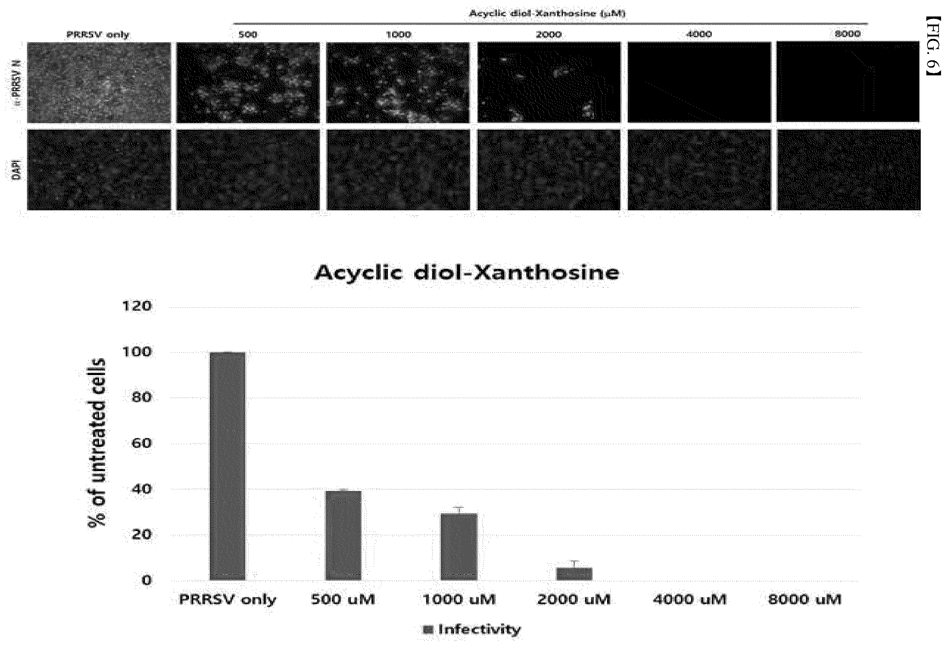

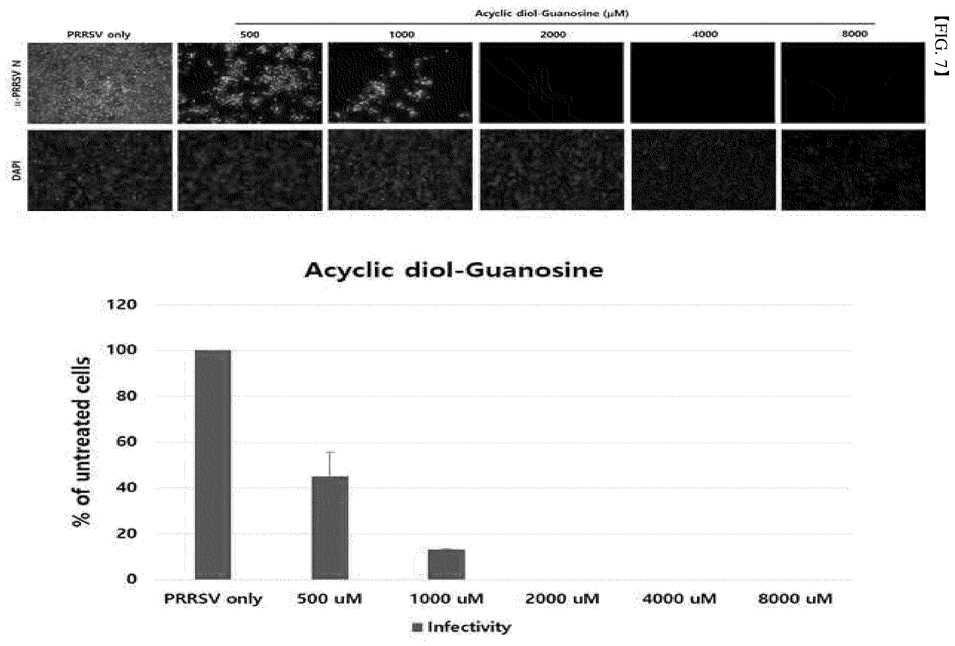

As shown in [FIG. 2], [FIG. 3], [FIG. 4], [FIG. 5], [FIG. 6] and [FIG. 7], as a result of evaluating the PRRSV infection and proliferation inhibition efficacy at each concentration of each material, in case of the guanosine in the dialdehyde form, infection inhibition was observed at a 50% level at a low concentration (40 µM), and infection inhibition of 90% or more was achieved at 100 µM, showing the best antiviral efficacy. As could be seen through DAPI staining at a concentration showing efficacy, as no cytotoxicity was observed even at up to 1000 µM, it was confirmed that the antiviral efficacy could be shown in a very safe concentration range. In addition, in the cytotoxicity evaluation using WST-1 experimental method, the cytotoxicity was not observed at 6000 or 10000 µM or more, so it was confirmed that the material was very safe [Table 1].

In conclusion, it was confirmed that the selectivity index shown in [Table 1] was also the highest in the guanosine in the dialdehyde form. The compound with efficacy second to the guanosine in the dialdehyde form was shown to be xanthosine in the dialdehyde form, and the inosine-based analogue showed efficacy at the highest concentration, but it was determined that the corresponding concentration was not at a high concentration level unsuitable for animal application. In addition, by treating inosine, xanthosine and guanosine in the acyclic diol form at a concentration of 500, 1000, 2000, 4000 and 8000 micromoles (µM), their efficacy levels and cytotoxicity were evaluated, and it was confirmed that the inosine, xanthosine and guanosine in the dialdehyde form had the antiviral efficacy against PRRSV at a higher concentration (IC₅₀: 400~500 µM).

**[Table 1]**

| PRRSV infection inhibition concentration, cytotoxicity and selectivity index by inosine, xanthosine and guanosine in dialdehyde form and acyclic diol form | | | |
|---|---|---|---|
| Material name | IC₅₀ | CC₅₀ | SI |
| Dialdehyde-inosine | 95 µM (±23) | > 10000 µM | > 105.3 |
| Dialdehvde-xanthosine | 75 µM (±8) | > 10000 µM | > 133.3 |
| Dialdehyde-guanosine | 40 µM (±6) | > 6000 µM | > 250 |
| Acyclic diol-inosine | 453.7 µM (±120.4) | > 8000 µM | > 17.6 |
| Acyclic diol-xanthosine | 399.8 µM (±4.8) | > 8000 µM | > 20 |
| Acyclic diol-guanosine | 457.85 µM (±60.35) | > 8000 µM | > 17.5 |

### Example 3: Verification of PED virus infection efficacy and safety based on in vitro cell evaluation

In order to evaluate the viral infection and proliferation inhibition efficacy against PED virus of inosine, xanthosine and guanosine in the dialdehyde form and acyclic diol form, monkey kidney cells (Vero cell line) were seeded in a 48 well plate at 4×10⁴ cells/well and then cultured for one day. The next day, PEDV (G1a type, SM98 strain) of 200 TCID₅₀/ml and inosine, xanthosine and guanosine in the dialdehyde and acyclic diol forms were treated to the cells simultaneously, and after 2 days, using commercialized Viral gene (DNA/RNA) extraction kit (iNtRON, 101410754), RNA was isolated. For the isolated RNA, using commercialized PEDV detection kit (Mediandiagnostics, NS-PED-31), the antiviral efficacy of the materials was verified.

As could be seen in [Table 2], the best efficacy was shown in guanosine in the dialdehyde form, and good effects were confirmed in the order of xanthosine and inosine in the dialdehyde form. In addition, the inosine, xanthosine and guanosine in the acyclic diol form also showed the antiviral efficacy against PEDV, and it showed IC₅₀ values at a higher concentration compared to the dialdehyde forms.

**[Table 2]**

| PEDV infection inhibition concentration, cytotoxicity and selectivity index by inosine, xanthosine and guanosine in dialdehyde form and acyclic diol form | | | |
|---|---|---|---|
| Classification | IC₅₀ | CC₅₀ | SI |
| Dialdehyde-inosine | 448.5 µM (±51.8) | > 10000 µM | > 22.3 |
| Dialdehyde-xanthosine | 398.5 µM (±160.9) | > 10000 µM | > 25.1 |
| Dialdehyde-guanosine | 156.6 µM (±8.2) | > 6000 µM | > 38.3 |
| Acyclic diol-inosine | 2767 µM (±219.2) | > 10000 µM | > 3.6 |
| Acyclic diol-xanthosine | 2296 µM (±12.7) | > 10000 µM | > 4.4 |
| Acyclic diol-guanosine | 1776 µM (±340.8) | > 10000 µM | > 5.6 |

### Example 4: Verification of infection efficacy against Porcine circovirus type 2 (PCV2) based on in vitro cell evaluation

In order to evaluate the viral infection and proliferation inhibition efficacy against PCV2 of inosine, xanthosine and guanosine in the dialdehyde form and acyclic diol form, pig kidney cells (PK15 cell line) were seeded in a 48 well plate at 4×10⁴ cells/well and then, PCV2 of 200 TCID₅₀/well and inosine, xanthosine and guanosine in the dialdehyde and acyclic diol forms were treated to the cells simultaneously, and after 4 days, using commercialized Viral gene (DNA/RNA) extraction kit (iNtRON, 101410754), DNA was isolated.

For the isolated DNA, using PCV2-specific primers (F-AGGAGGGCGTTCTGACTGTG, R-ACCGCTACCGTTGGAGAAGG) and realtime RT-qPCR experimental method (95°C, 30 seconds 1 cycle, 95°C, 20 seconds, 56°C, 60 seconds 40 cycles), the antiviral efficacy of the materials was verified.

As could be seen in [Table 3], the overwhelmingly good efficacy was shown in guanosine in the dialdehyde form, and good effects were confirmed in the order of xanthosine and inosine in the dialdehyde form. In addition, the inosine, xanthosine and guanosine in the acyclic diol form also showed the antiviral efficacy against PCV2, and guanosine in the acyclic diol form showed an IC₅₀ value at a relatively higher concentration.

**[Table 3]**

| PCV2 infection inhibition concentration, cytotoxicity and selectivity index by inosine, xanthosine and guanosine in dialdehyde form and acyclic diol form | | | |
|---|---|---|---|
| Classification | IC₅₀ | CC₅₀ | SI |
| Dialdehyde-inosine | 817.2 µM (±844) | > 10000 µM | > 12.2 |
| Dialdehyde-xanthosine | 435.6 µM (±154.4) | > 10000 µM | > 23 |
| Dialdehvde-guanosine | 96.6 µM (±26.4) | > 6000 µM | > 62.1 |
| Acyclic diol-inosine | 776.4 µM (±166.5) | > 10000 µM | > 12.9 |
| Acyclic diol-xanthosine | 994.9 µM (±227.8) | > 10000 µM | > 10.1 |
| Acyclic diol-guanosine | 2334 µM (±390.3) | > 10000 µM | > 4.3 |

### Example 5: Verification of Porcine rotavirus (PRY) infection efficacy and safety based on in vitro cell evaluation

In order to evaluate the viral infection and proliferation inhibition efficacy against PRV of inosine, xanthosine and guanosine in the dialdehyde form and acyclic diol form, monkey kidney cells (Vero cell line) were seeded in a 48 well plate at 4×10⁴ cells/well and then cultured for one day. The next day, PEDV (G1a type, SM98 strain) of 200 TCID₅₀/ml and inosine, xanthosine and guanosine in the dialdehyde and acyclic diol forms were treated to the cells simultaneously, and after 3 days, using commercialized Viral gene (DNA/RNA) extraction kit (iNtRON, 101410754), RNA was isolated. For the isolated RNA, using commercialized PRV detection kit (Ingenetix, DHUV02053), the antiviral efficacy of the materials was verified.

As could be seen in [Table 4], in inosine and xanthosine in the dialdehyde form, the antiviral efficacy at a similar level (forming IC₅₀ values at a concentration of 100 µM or less) was shown, and in guanosine, the IC₅₀ value at a concentration of 177.4 µM were shown. In addition, in the acyclic diol forms, the IC₅₀ values at a higher concentration than the materials in the dialdehyde forms were shown, and the efficacy was observed in the order of inosine, guanosine and xanthosine.

**[Table 4]**

| PRV infection inhibition concentration, cytotoxicity and selectivity index by inosine, xanthosine and guanosine in dialdehyde form and acyclic diol form | | | |
|---|---|---|---|
| Classification | IC₅₀ | CC₅₀ | SI |
| Dialdehyde-inosine | 96.6 µM (±9.3) | > 10000 µM | > 103.5 |
| Dialdehvde-xanthosine | 90.2 µM (±13.4) | > 10000 µM | > 110.9 |
| Dialdehvde-guanosine | 177.4 µM (±0.9) | > 6000 µM | > 33.8 |
| Acyclic diol-inosine | 2832.5 µM (±317.5) | > 10000 µM | > 3.5 |
| Acyclic diol-xanthosine | 3420 µM (±446.9) | > 10000 µM | > 2.9 |
| Acyclic diol-guanosine | 3399.5 µM (±536.7) | > 10000 µM | > 2.9 |

### Example 6: Verification of Influenza virus (IV) infection efficacy and safety based on in vitro cell evaluation

In order to evaluate the viral infection and proliferation inhibition efficacy against Swine Influenza virus (SIV) of inosine, xanthosine and guanosine in the dialdehyde form and acyclic diol form, dog kidney cells (MDCK cell line) were seeded in a 48 well plate at 5×10⁴ cells/well and then cultured for one day. The next day, SIV of 200 TCID₅₀/well and inosine, xanthosine and guanosine in the dialdehyde and acyclic diol forms were treated to the cells simultaneously, and after 4 days, using commercialized Viral gene (DNA/RNA) extraction kit (iNtRON, 101410754), RNA was isolated.

For the isolated RNA, using SIV-specific primers (F-GATGGTAGATGGATGGTACGGTTAT, R-TTGTTAGTAATCTCGTCAATGGCATT) and realtime RT-qPCR experimental method (95°C, 10 minutes 1 cycle, 95°C, 15 seconds, 60°C, 30 seconds, 72°C, 30 seconds 40 cycles), the antiviral efficacy of the materials was verified.

As could be seen in [Table 5], it could be confirmed that the infection and proliferation of SIV were inhibited, when inosine, xanthosine and guanosine in the dialdehyde form and acyclic diol dorm were treated into the cells. Guanosine in the dialdehyde form showed the most excellent efficacy (IC₅₀: 171.3 µM), and good effects were confirmed in the order of inosine and xanthosine. In addition, even in the acyclic diol forms, the antiviral efficacy could be confirmed, but the efficacy was confirmed at a very high concentration.

**[Table 5]**

| SIV infection inhibition concentration, cytotoxicity and selectivity index by inosine, xanthosine and guanosine in dialdehyde form and acyclic diol form | | | |
|---|---|---|---|
| Classification | IC₅₀ | CC₅₀ | SI |
| Dialdehvde-Inosine | 554 µM (±212.9) | > 10000 µM | > 18.1 |
| Dialdehvde-Xantho sine | 748.2 µM (±384.5) | > 10000 µM | > 13.4 |
| Dialdehyde-Guanosine | 171.3 µM (±119.7) | > 6000 µM | > 35 |
| Acyclic diol-Inosine | 6193 µM (±3074.5) | > 10000 µM | > 1.6 |
| Acyclic diol-Xanthosine | 6693.5 µM (±3530.6) | > 10000 µM | > 1.5 |
| Acyclic diol-Guanosine | 4816.5 µM (±2030.1) | > 10000 µM | > 2.1 |

### Example 7: Verification of infection efficacy and safety against Avian Infectious Bronchitis virus (IBV) based on in vitro cell evaluation

In order to evaluate the viral infection and proliferation inhibition efficacy against IBV of inosine, xanthosine and guanosine in the dialdehyde form and acyclic diol form, monkey kidney cells (Vero cell line) were seeded in a 48 well plate at 4×10⁴ cells/well and then cultured for one day. The next day, IBV of 200 TCID₅₀/well and inosine, xanthosine and guanosine in the dialdehyde and acyclic diol forms were treated to the cells simultaneously, and after 4 days, using commercialized Viral gene (DNA/RNA) extraction kit (iNtRON, 101410754), RNA was isolated.

For the isolated RNA, using IBV-specific primers (F-ATGCTCAACCTTGTCCCTAGCA, R-TCAAACTGCGGATCATCACGT) and realtime RT-qPCR experimental method (95°C, 10 minutes 1 cycle, 95°C, 15 seconds, 60°C, 45 seconds, 72°C, 30 seconds 40 cycles), the antiviral efficacy of the materials was evaluated.

As could be seen in [Table 6], the infection and proliferation inhibition efficacy of IBV by inosine, xanthosine and guanosine in the dialdehyde form and acyclic diol form could be confirmed. Inosine in the dialdehyde form showed the best efficacy (IC₅₀: 76.4 µM), and the antiviral effects were confirmed in the order of xanthosine and guanosine. In addition, even in the acyclic diol forms, xanthosine and guanosine showed IC₅₀ values at a similar level, and compared thereto, inosine showed the efficacy at a relatively high IC₅₀ value.

**[Table 6]**

| IBV infection inhibition concentration, cytotoxicity and selectivity index by inosine, xanthosine and guanosine in dialdehyde form and acyclic diol form | | | |
|---|---|---|---|
| Classification | IC₅₀ | CC₅₀ | SI |
| Dialdehvde-Inosine | 76.4 µM (±2.2) | > 10000 µM | > 130 |
| Dialdehyde-Xanthosine | 225.8 µM (±21.7) | > 10000 µM | > 44.3 |
| Dialdehyde-Guanosine | 274.9 µM (±194.9) | > 6000 µM | > 21.8 |
| Acyclic diol-Inosine | 2663.5 µM (±1048.6) | > 10000 µM | > 3.8 |
| Acyclic diol- | 1799 µM (±302.6) | > 10000 µM | > 5.6 |
| Acyclic diol-Guanosine | 1775 µM (±188.1) | > 10000 µM | > 5.6 |

### Example 8: Verification of Avian Newcastle virus (NDV) infection efficacy and safety based on in vitro cell evaluation

In order to evaluate the viral infection and proliferation inhibition efficacy against NDV of inosine, xanthosine and guanosine in the dialdehyde form and acyclic diol form, monkey kidney cells (Vero cell line) were seeded in a 48 well plate at 4×10⁴ cells/well and then cultured for one day. The next day, NDV of 200 TCID₅₀/well and inosine, xanthosine and guanosine in the dialdehyde and acyclic diol forms were treated to the cells simultaneously, and after 3 days, using commercialized Viral gene (DNA/RNA) extraction kit (iNtRON, 101410754), RNA was isolated. For the isolated RNA, using commercialized NDV detection kit (iNtRON, IP15456), the antiviral efficacy of the materials was verified.

As could be seen in [Table 7], inosine in the dialdehyde form showed the most excellent efficacy (IC₅₀: 469.4 µM), and the viral infection and proliferation efficacy was confirmed in the order of xanthosine and guanosine. Inosine and guanosine in the acyclic diol form showed the antiviral efficacy at a higher IC₅₀ value compared to the materials in the dialdehyde form, and xanthosine showed the NDV infection inhibition efficacy of 39.4% (±0.97) at a concentration of 8000 µM.

**[Table 7]**

| NDV infection inhibition concentration, cytotoxicity and selectivity index by inosine, xanthosine and guanosine in dialdehyde form and acyclic diol form | | | |
|---|---|---|---|
| Classification | IC₅₀ | CC₅₀ | SI |
| Dialdehyde-Inosine | 469.4 µM (±13.3) | > 10000 µM | > 21.3 |
| Dialdehyde-Xanthosine | 525.6 µM (±6.7) | > 10000 µM | > 19 |
| Dialdehyde-Guanosine | 1119.2µM (±462.2) | > 6000 µM | > 5.4 |
| Acyclic diol-Inosine | 4442.5 µM (±645.6) | > 10000 µM | > 2.3 |
| Acyclic diol-Xanthosine | 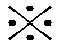 At 8000 µM, showing NDV infection inhibition efficacy of 39.4% (±0.97) | | |
| Acyclic diol-Guanosine | 6712 µM (±3627.5) | > 10000 µM | > 1.5 |

### Example 9: Verification of infection efficacy and safety against Bovine Viral Diarrhea virus (BVD) based on in vitro cell evaluation

In order to evaluate the viral infection and proliferation inhibition efficacy against BVDV of inosine, xanthosine and guanosine in the dialdehyde form and acyclic diol form, bovine kidney cells (MDBK cell line) were seeded in a 48 well plate at 4×10⁴ cells/well and then cultured for one day. The next day, BVDV of 200 TCID₅₀/well and inosine, xanthosine and guanosine in the dialdehyde and acyclic diol forms were treated to the cells simultaneously, and after 4 days, using commercialized Viral gene (DNA/RNA) extraction kit (iNtRON, 101410754), RNA was isolated.

For the isolated RNA, using BVDV-specific primers (F-TGACACCATCACCGACCAC, R-CTCCCTCTCTGCCCATTTCTT) and realtime RT-qPCR experimental method (94°C, 10 minutes 1 cycle, 94°C, 10 seconds, 53°C, 20 seconds, 72°C, 30 seconds 35 cycles), the antiviral efficacy of the materials was verified.

As could be seen in [Table 8], it could be confirmed that the infection and proliferation of BVDV were inhibited, when inosine, xanthosine and guanosine in the dialdehyde form and acyclic diol form were treated into the cells. Xanthosine in the dialdehyde form showed the most excellent efficacy (IC₅₀: 97.8 µM), and good effects were confirmed in the order of inosine and guanosine. In addition, in the acyclic diol forms, the efficacy could be confirmed at a higher IC₅₀ value compared to the dialdehyde form (confirmed virus titer reduction efficacy of log₁₀ 1 or more).

**[Table 8]**

| BVDV infection inhibition concentration, cytotoxicity and selectivity index by inosine xanthosine and guanosine in dialdehyde form and acyclic diol form | | | |
|---|---|---|---|
| Classification | IC₅₀ | CC₅₀ | SI |
| Dialdehyde-inosine | 110.2 µM (±9.3) | > 10000 µM | > 90.8 |
| Dialdehyde-xanthosine | 97.8 µM (±2.9) | > 10000 µM | > 102.2 |
| Dialdehyde-guanosine | 2757µM (±241.8) | > 6000 µM | > 2.2 |
| Acyclic diol- | 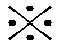 At 8000 µM, showing BVDV infection inhibition efficacy of 18.9% (±3.28) | | |
| Acyclic diol- | 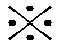 At 8000 µM, showing BVDV infection inhibition efficacy of 30.4% (±0.87) | | |
| Acyclic diol- | 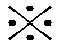 At 8000 µM, showing BVDV infection inhibition efficacy of 24.3% (±10.29) | | |

### Example 10: Verification of infection efficacy and safety against Bovine rotavirus (BRV) based on in vitro cell evaluation

In order to evaluate the viral infection and proliferation inhibition efficacy against BRV of inosine, xanthosine and guanosine in the dialdehyde form and acyclic diol form, bovine kidney cells (MDBK cell line) were seeded in a 48 well plate at 4×10⁴ cells/well and then cultured for one day. The next day, BRV of 200 TCID₅₀/well and inosine, xanthosine and guanosine in the dialdehyde and acyclic diol forms were treated to the cells simultaneously, and after 4 days, using commercialized Viral gene (DNA/RNA) extraction kit (iNtRON, 101410754), RNA was isolated.

For the isolated RNA, using BRV-specific primers (F-TCATTTCAGTTGATGAGACCACC, R-ATTCAATTCTAAGCGTGAGTCCTAC) and realtime RT-qPCR experimental method (95°C, 10 minutes 1 cycle, 95°C, 15 seconds, 60°C, 15 seconds, 72°C, 20 seconds 40 cycles), the antiviral efficacy of the materials was confirmed.

As could be seen in [Table 9], xanthosine in the dialdehyde form showed very excellent infection and proliferation inhibition efficacy against BRV (IC₅₀: 50.5 µM), and excellent effects could be confirmed in the order of inosine and guanosine. The acyclic diol forms showed the antiviral efficacy against BRV in the order of xanthosine, guanosine and inosine, but showed a slightly higher IC₅₀ value compared to the dialdehyde forms.

**[Table 9]**

| BRV infection inhibition concentration, cytotoxicity and selectivity index by inosine, xanthosine and guanosine in dialdehyde form and acyclic diol form | | | |
|---|---|---|---|
| Classification | IC₅₀ | CC₅₀ | SI |
| Dialdehvde-Inosine | 106.6 µM (±13.3) | > 10000 µM | > 93.8 |
| Dialdehyde-Xanthosine | 50.5 µM (±10.7) | > 10000 µM | > 198.1 |
| Dialdehyde-Guanosine | 88.3 µM (±16.2) | > 6000 µM | > 68 |
| Acyclic diol-Inosine | 1931 µM (±147.8) | > 10000 µM | > 5.2 |
| Acyclic diol-Xanthosine | 1561 µM (±15.6) | > 10000 µM | > 6.4 |
| Acyclic diol-Guanosine | 11761.5 µM (±17.7) | > 10000 µM | > 5.7 |

### Example 11: Verification of infection efficacy and safety against Canine Parvovirus (CPV) based on in vitro cell evaluation

In order to evaluate the viral infection and proliferation inhibition efficacy against CPV of inosine, xanthosine and guanosine in the dialdehyde form and acyclic diol form, dog kidney cells (MDCK cell line) were seeded in a 48 well plate at 5×10⁴ cells/well and then CPV of 200 TCID₅₀/well and inosine, xanthosine and guanosine in the dialdehyde form and acyclic diol form were treated to the cells simultaneously, and after 4 days, using commercialized Viral gene (DNA/RNA) extraction kit (iNtRON, 101410754), DNA was isolated.

For the isolated DNA, using CPV-specific primers (F-AGCTAGTTTCATCTTAACTATCAAATT, R-TTAACTCTCCAGAAAACTCATGC) and realtime RT-qPCR experimental method (95°C, 5 minutes 1 cycle, 95°C, 10 seconds, 60°C, 30 seconds, 40 cycles), the antiviral efficacy of the materials was confirmed.

As could be seen in [Table 10], it could be confirmed that the infection and proliferation of CPV were inhibited, when inosine, xanthosine and guanosine in the dialdehyde form and acyclic diol form were treated into the cells. In xanthosine in the dialdehyde form, the most excellent efficacy (IC₅₀: 221.2 µM) was observed, followed by inosine and guanosine. In case of the acyclic diol forms, inosine and xanthosine showed the antiviral efficacy of almost similar IC₅₀ values (2194 and 2195.5 µM), and guanosine showed the CPV infection inhibition efficacy of 18.0% (±0.68) at 8000 µM (confirmed virus titer reduction efficacy of log₁₀ 1 or more).

**[Table 10]**

| PCV2 infection inhibition concentration, cytotoxicity and selectivity index by inosine, xanthosine and guanosine in dialdehyde form and acyclic diol form | | | |
|---|---|---|---|
| Classification | IC₅₀ | CC₅₀ | SI |
| Dialdehyde-Inosine | 1689.7 µM (±2352.2) | > 10000 µM | > 5.9 |
| Dialdehyde-Xanthosine | 221.2 µM (±259.1) | > 10000 µM | > 45.2 |
| Dialdehyde-Guanosine | 2055.5 µM (±2798) | > 6000 µM | > 2.9 |
| Acyclic diol-Inosine | 2194 µM (±417.2) | > 10000 µM | > 4.6 |
| Acyclic diol-Xanthosine | 2195.5 µM (±828) | > 10000 µM | > 4.6 |
| Acyclic diol-Guanosine | 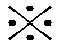 At 8000 µM, showing CPV infection inhibition efficacy of 18.0% (±0.68) | | |

### Example 12: Verification of infection efficacy and safety against Influenza virus (IV) based on in vivo mouse challenge evaluation

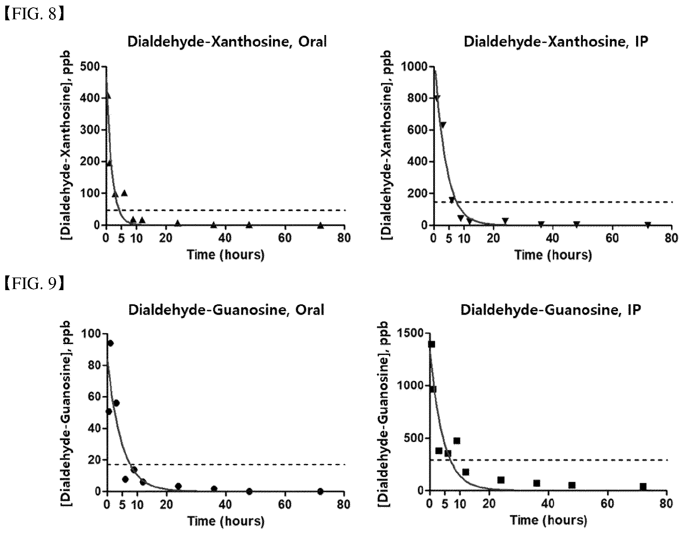

To analyze in vivo efficacy of analogues of xanthosine and guanosine in the dialdehyde form using mice, before evaluation of challenge using Influenza virus, to determine the inoculation method and frequency and concentration and the like, Pharmaco-kinetics (PK) analysis in an informal form was performed. When comparing the two kinds of inoculation methods, one was analyzing the change in concentration of the materials in the blood through 10 blood samplings after compulsory oral inoculation of 200 ul into mice at a concentration of 250 mg/kg through an intragastric route. The other inoculation method was performing analysis after blood sampling in the same method after inoculating 100 ul through a syringe at a concentration of 50 mg/kg through an intragastric route. In [FIG. 8] and [FIG. 9], the residual concentration values at 10 time points were shown and they were analyzed by drawing a trend line, and as a result of estimating the point in time when the material concentration was halved, based on Area under the curve (AUC) values, for guanosine in the dialdehyde form, about 9 hours in case of intragastric route inoculation, and about 6 hours in case of intraperitoneal route inoculation were times when 50% of the remaining materials was measured, and for xanthosine in the dialdehyde form, in both the intragastric route and intraperitoneal route, it showed about 6 hours all. The half-life of dropping to the half or less at the Max concentration in plasma was shown to be 1.3~3.1 hours, and in the dialdehyde xanthosine with slightly high water solubility, the half-life tended to be shorter. Comparing the Max concentrations, it was shown to be higher in the intraperitoneal injection administration than in oral administration, and based on this, it could be seen that a mechanism to increase an absorption rate may be required in the oral administration.

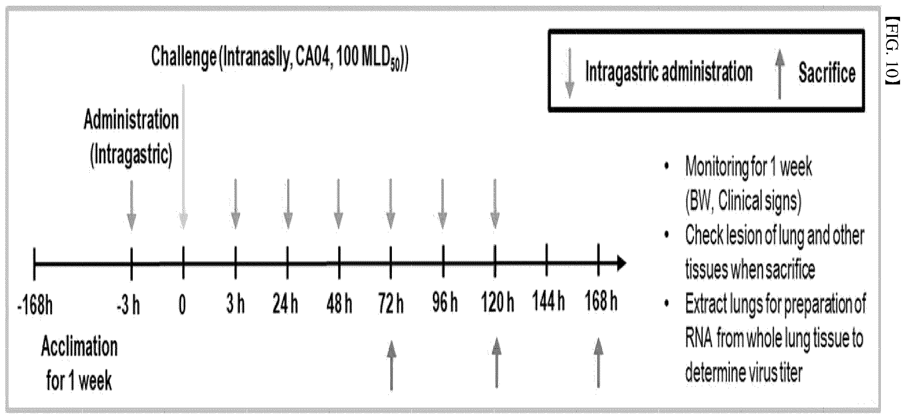

Referring to the above result, a material inoculation plan was established during challenge evaluation of Influenza virus (2009 pandemic strain, CA04 (virus isolated from a patient infected with a swine-derived influenza virus)) using mice, and the design and schedule of the test treatment groups were shown in [FIG. 10] and [Table 11].

**[Table 11]**

| Treatment groups, schedule and test information of in vivo Influenza virus challenge test using experimental animals (mice) | | | | |
|---|---|---|---|---|
| Grou P | Treatment | SIV challenge | Number of objects per group | Inoculation Route |
| NC | PBS 20*µ*ℓ volume | CA04 30*µ*ℓ volume (PBS, IN) | 7 | Intragastric |
| T1 | Dialdehyde-Guanosine 250 mg/kg, 200*µ*ℓ volume (PBS) | | 7 | Intragastric |
| T2 | Dialdehyde-Xanthosine 250 mg/kg, 200*µ*ℓ volume (PBS) | | 7 | Intragastric |

### Test contents

- Lung tissue extraction by Time Point (Virus titration)
- Observation of lung tissue lesions during autopsy
- Observation of daily clinical symptoms
- Observation of damage by organ tissue during autopsy
- Confirmation of daily body weight
- 3, 5 dpi, autopsy of 1 animal per treatment group (upon completion of 7 dpi test, full autopsy)

As shown in [FIG. 10], after the acclimation period of 1 week, at 3 hours before and after challenge of SIV (2009 pandemic strain CA04) of 100 MLD₅₀ (50% mouse lethal dose) through an intranasal route, oral (intragastric) administration was performed by the inoculation method shown in [Table 11], and administration of the materials was performed once a day up to the 5th day after the virus challenge. Monitoring was conducted up to the 7th day of performing the final autopsy, and during the period of performing, clinical symptom observation and body weight (BW) were measured every day. On the 3rd day and 5th day after infection, one animal by each treatment group was autopsied, and lung lesions by the virus were observed, and abnormal lesions in the liver, stomach, spleen, kidney, heart, small intestine, and large intestine were observed, to perform verification of safety of the materials. Total RNA was extracted from the extracted lung hereafter and the remaining amount of the virus in lung tissue was traced to measure the efficacy of the antiviral materials.

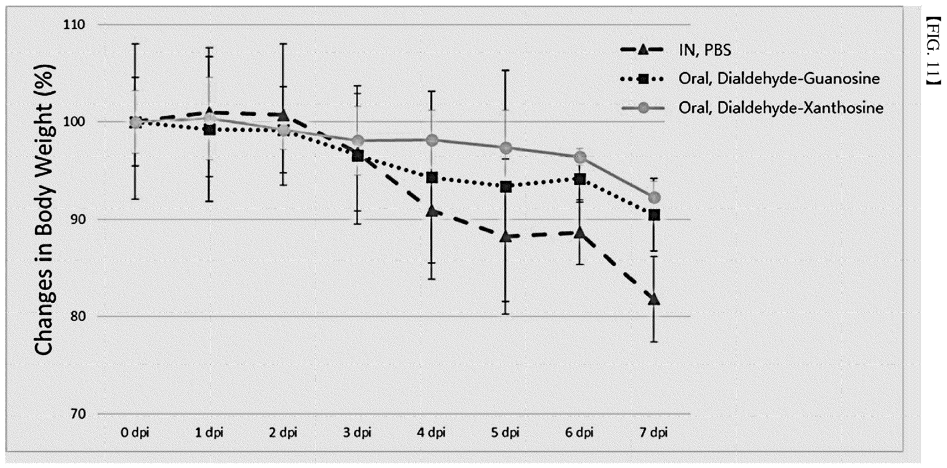

As a result, as confirmed in vitro, in vivo Anti-Influenza virus efficacy of orally administered xanthosine and guanosine in the dialdehyde was shown, and abnormal lesions due to fed materials were not observed, and thus, the safety was also verified. However, compared to the control group (the group fed with only PBS (phosphate buffered saline)), the efficacy was excellent, but a difference in efficacy was shown between the two groups of xanthosine and guanosine in the dialdehyde form, and the absorption rate of xanthosine in the dialdehyde form with high solubility was good, and therefore, contrary to the cell-based in vitro antiviral efficacy evaluation, the efficacy was mor excellent than guanosine in the dialdehyde form. To explain in detail, as could be seen in [FIG. 11], as a result of observing the change in body weight during the SIV challenge evaluation for 7 days, mice administered with only PBS showed weight loss due to the virus from the 3rd day, and the mice administered intragastrically (method for oral inoculation) with analogues of xanthosine and guanosine in the dialdehyde form showed only very mild symptoms during the period while the materials were administered up to the 5th day and the materials were remained in the body until the 6th day, and the weight loss was also very slight, but on the 7th day when the materials were not fed for 2 days, weight loss was observed. However, as the level of weight loss was slight, it could be confirmed that the antiviral efficacy according to oral feeding of the materials until the end of the test was very excellent, compared to the control group. By comprehensively analyzing the rate of change in weight loss, information that material feeding should be administered until the virus is completely eradicated could be obtained, and overall, it could be proven that feeding of the materials had an effect of minimizing viral symptoms.

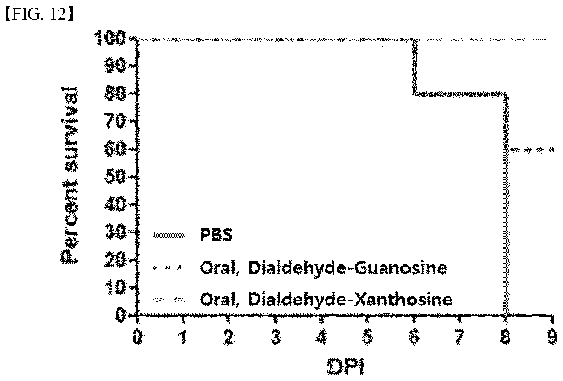

The survival rate could be explained based on the above result, as shown in [FIG. 12], as a result of predicting the survival rate result on the 8th day based on all data up to the 7th day, the result that the control group in which weight loss was very severe showed 100% mortality (80% survival rate on the 7th day), and the groups orally fed with xanthosine and guanosine in the dialdehyde form showed similarly to the weight loss pattern, and the group of xanthosine in the dialdehyde form showed 100% survival (100% survival rate on the 7th day), and the group of guanosine in the dialdehyde form showed 60% survival rate (80% survival rate on the 7th day). It could be assumed that the same result was shown in the weight change and survival rate, since the xanthosine material in the dialdehyde form with a high absorption rate (See PK result) due to its high water solubility showed that the concentration in plasma was high.

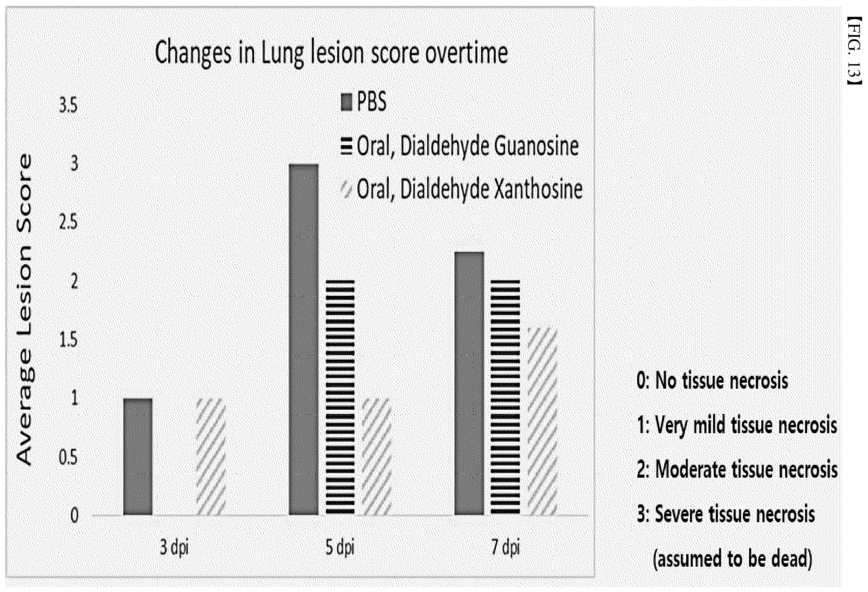

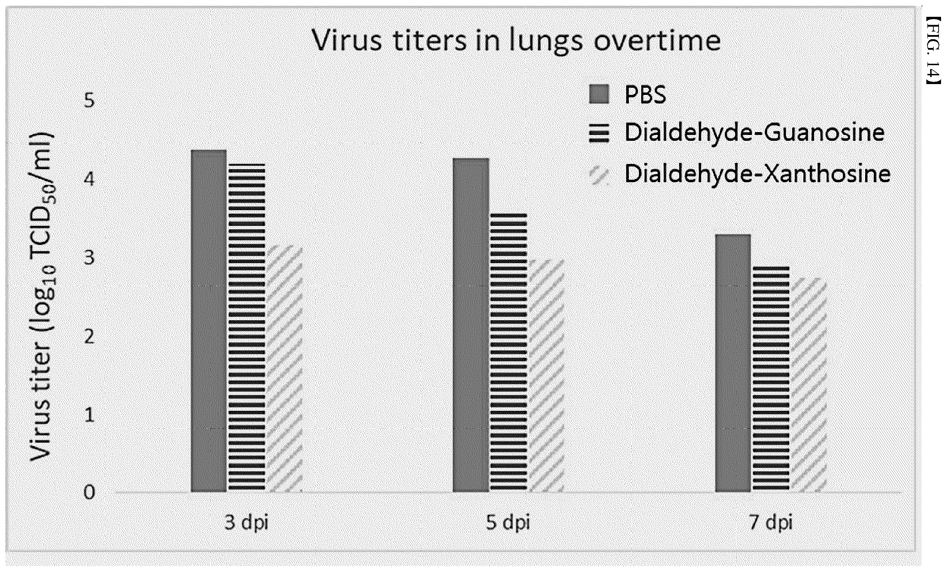

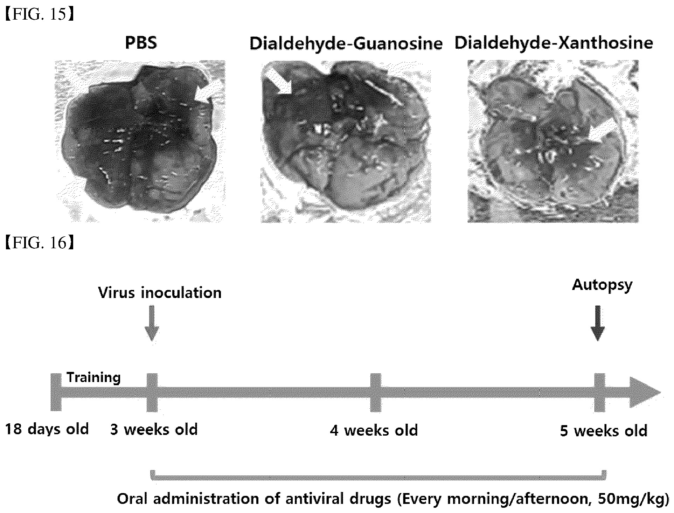

Also, in observation of lung lesions through autopsy, the results in the same pattern as above were observed, and as could be seen in [FIG. 13], it was shown that the lesions on the 3rd day, when virus proliferation peaked, were mild, but the lung lesions on the 5^{th} day was very serious due to the aftereffect of virus proliferation. On the other hand, xanthosine and guanosine in the dialdehyde form showed the same result as the clinical symptom result observed in appearance, as mild lung lesions were observed, compared to the control group. In the same context, there is a part shown in a condition in which the gap of the symptom level with the control group was greatly narrowed on the 7th day, when the lung lesions were observed, in that feeding of the materials was not carried out, after the 5th day, and the result of virus complete eradication could not be seen due to the short evaluation period of 7 days. Nevertheless, by comparing and analyzing the observation result on the 5th day, it can be said that the antiviral efficacy of the fed materials can be clearly confirmed. In addition, as shown in [FIG. 14], when the materials were fed, the result that the amount of the virus remaining in the lung tissue was measured slightly lower could be confirmed, and in xanthosine in the dialdehyde form showing more excellent efficacy than guanosine in the dialdehyde form, the lowest virus titer could be confirmed. As a result, it can be said that the viral proliferation by the materials was inhibited, and thus, the clinical symptoms and autopsy results were derived as above. Additionally, [FIG. 15] are photographs of extracted lung tissue showing representative lung lesions in each group at autopsy, and while the control group showed fully filling blood components or tissue necrosis at a very severe level, only the lesions at a mild level compared to the control group were observed in the xanthosine and guanosine in the dialdehyde form, and thus it can be said to be a visual observation result capable of supporting the above result. In order to confirm the actual effect on a specific virus for each livestock species, the material dosage or frequency or the like should be newly derived for each livestock species targeted by the virus, and information on the duration of administration should be obtained, but in vivo, xanthosine and guanosine in the dialdehyde form effectively showed the antiviral efficacy (alleviation of disease symptoms and inhibition of viral proliferation), and therefore, it can be said that the basis for claiming the antiviral efficacy for each virus claimed in the in vitro example shown above by that the in vivo efficacy could be preferentially confirmed.

For reference, the influenza virus used in the present pre-clinical evaluation using small animals is a virus that can infect both chickens and pigs and exhibit disease symptoms, and it can be said that they can be used as a therapeutic agent for an antiviral use in chickens and pigs, as the disease symptoms are alleviated and the survival rate increases by xanthosine and guanosine in the dialdehyde form, when chickens and pigs were infected with Influenza virus.

### Example 13: Verification of infection efficacy and safety against Porcine reproductive and respiratory syndrome (PRRS) virus based on in vivo piglet challenge evaluation

To analyze the in vivo antiviral efficacy of XMP in a dialdehyde form using young pig (piglets), challenge evaluation using PRRS virus was performed, and an isolator for preventing airborne spread of PRRSV was used, and to secure discrimination of virus clinical symptoms, 3-week-old piglets were used. As described in [Table 12], the treatment groups were arranged, and as described in [FIG. 16], evaluation was conducted. 18-week-old piglets were stood and after the acclimation period of 3 days, PRRSV (NA strain, highly pathogenic than EU strain) was challenged through an intranasal route, and the materials or PBS (Phosphate buffered saline) were orally fed using a catheter every morning and afternoon. The body weight was measured at intervals of 3-4 days after challenge, and monitoring of clinical symptoms was performed. In 2 weeks after the start of the challenge, through autopsy, lesions in lung tissue were confirmed to verify the antiviral efficacy of xanthosine in the dialdehyde form.

**[Table 12]**

| Arrangement of treatment groups for evaluation of antiviral efficacy in PRRSV-infected piglets | | | | |
|---|---|---|---|---|
| Group | Treatment | PRRSV challenge | Number of piglets | Inoculation Route |
| Negative control group | - | NA strain 2ml volume (PBS, IN) | 8 (4 Female, 4 Male) | - |
| Positive control group | PBS, 5ml volume | | 8 (4 Female, 4 Male) | Oral (twice/day) |
| Material treatment group | Dialdehyde-Xanthosine 50 mg/kg/day, 5ml volume (PBS) | | 8 (4 Female, 4 Male) | Oral (twice/day) |

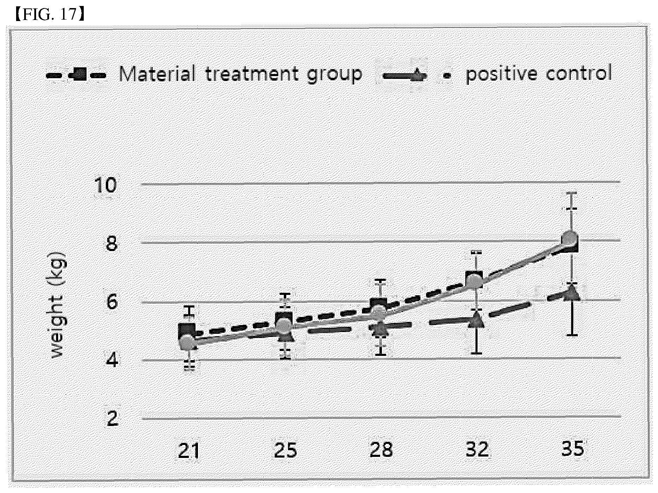

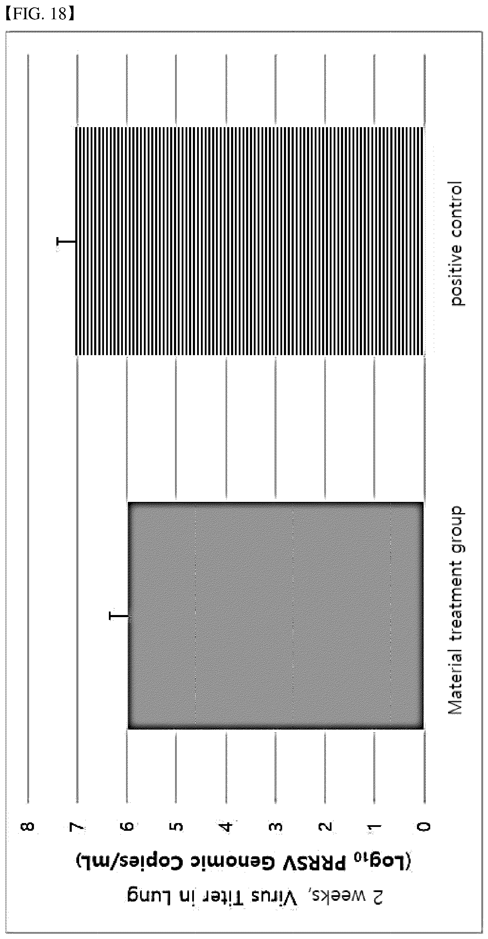

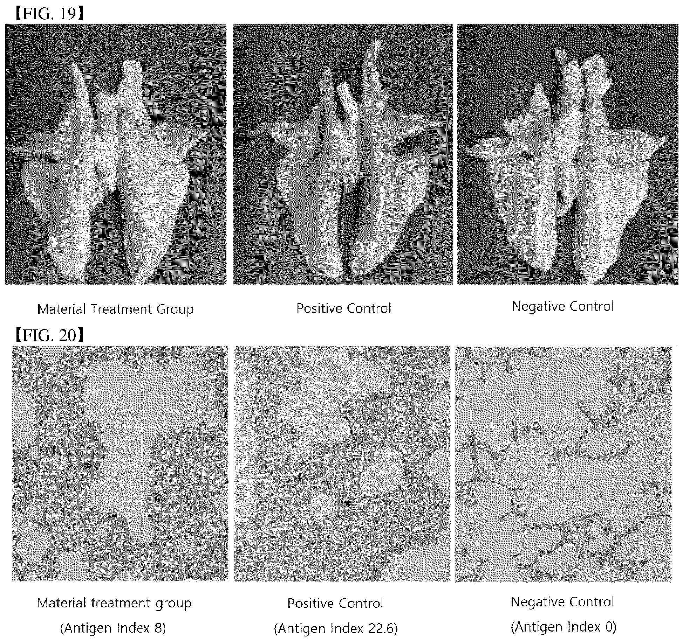

As shown in [FIG. 17], in the positive control group in which virus inoculation and PBS feeding were carried out, due to damage by virus infection, the weight gain progressed very slowly, but the weight gain of the piglets orally fed with the materials showed no delay in weight gain and showed the same weight gain pattern as the positive control group to which any treatment was not carried out at all. This is a very important result, and it is a fact that economic losses due to PRRSV infection can be prevented in actual livestock farms, when xanthosine in the dialdehyde form was fed with feed. In addition, as a result of measuring a virus titer in lung tissue (tropism of PRRSV is macrophages present in the lung) during autopsy, as shown in [FIG. 18], the amount of virus reduced compared to the positive control group could be confirmed in the material treatment group. This demonstrates that the efficacy of alleviating clinical symptoms shows the antiviral efficacy by actually reducing the remaining amount of virus in the host body and inhibiting viral proliferation. [FIG. 19] is photographs of visually observing interstitial pneumonia lesions by extracting lung during autopsy, and the negative control group and material treatment group shows lung in a relatively intact lung, unlike to the positive control group showing lesions throughout the lung. Finally, immunohistochemistry was performed using an antibody specifically detecting PRRS virus by peeling and sectioning the extracted lung, and as shown in [FIG. 20], unlike the positive control group in which numerous viruses were present, in the material treatment group, only a very small amount of viruses were detected. To summarize all the experimental results, it can be determined that PRRS virus infectious disease chronically causing economic damage in pig farms can be treated through oral feeding of xanthosine in the dialdehyde form.

### [SEQUENCE LISTING]

Sequence listing electronic file attached
(C:\KipoNet\NKEditor\appfile\DPP20222746IPPKS_220812.xml)

## Claims

1. An antiviral composition comprising at least one nucleoside analogue selected from the group consisting of nucleoside analogues of the following Chemical formulas 1 to 6 and pharmaceutically acceptable salts thereof:

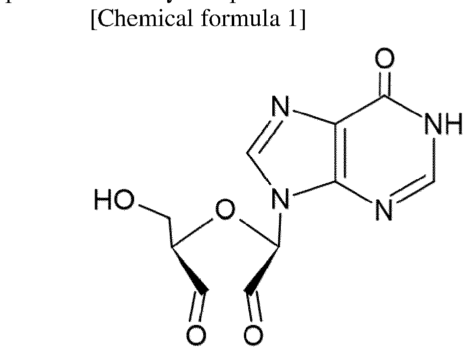

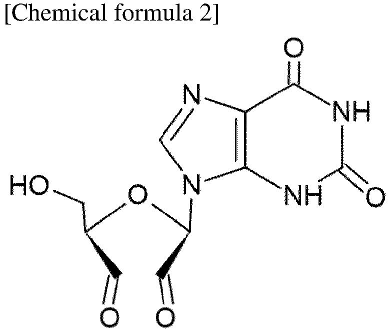

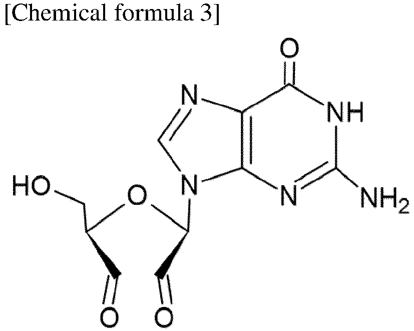

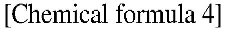

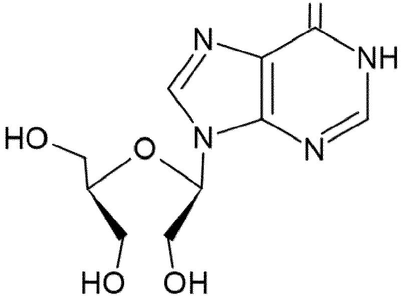

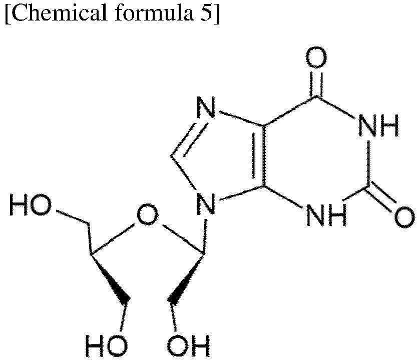

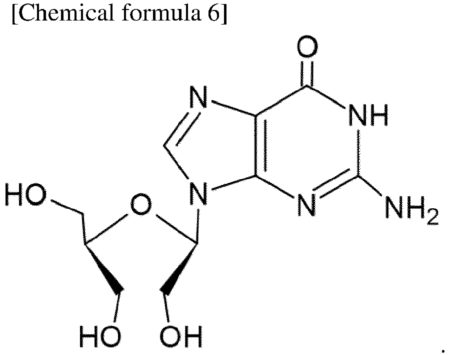

2. The composition according to claim 1, wherein the virus may be at least one virus selected from the group consisting of Porcine Reproductive and Respiratory Syndrome virus (PRRSV), Porcine Epidemic Diarrhea virus (PEDV), Influenza virus (Swine Influenza virus, SIV, Avian Influenza virus, AIV), Porcine Rotavirus (PRV), Porcine Circovirus (PCV), Avian Infectious Bronchitis virus (IBV), Avian Newcastle Disease virus (NDV), Bovine Viral Diarrhea virus (BVDV), Bovine Rotavirus (BRV) and Canine Parvovirus (CPV).

3. An immunomodulatory composition comprising at least one nucleoside analogue selected from the group consisting of nucleoside analogues of the following Chemical formulas 1 to 6 and pharmaceutically acceptable salts thereof:

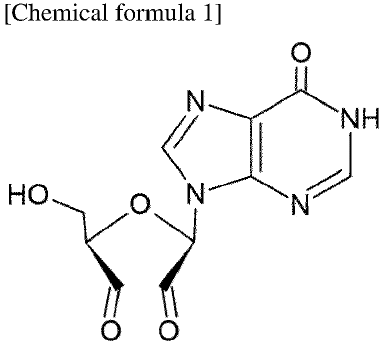

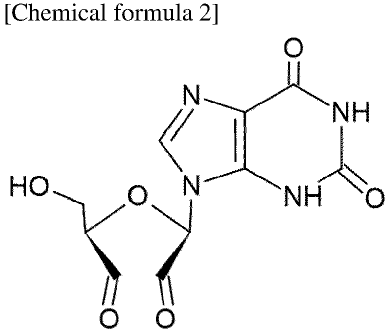

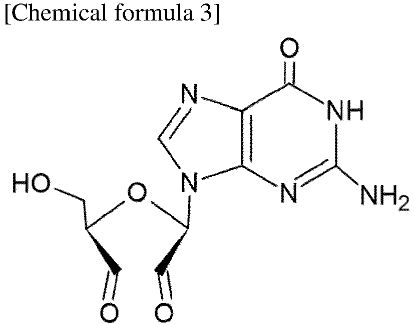

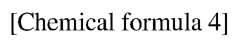

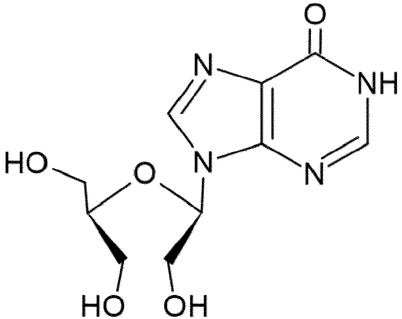

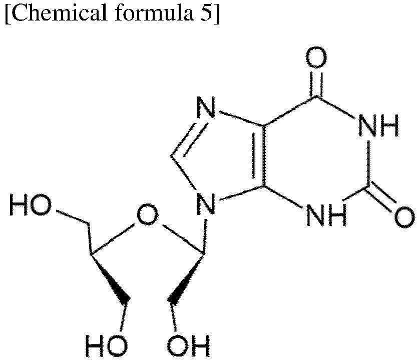

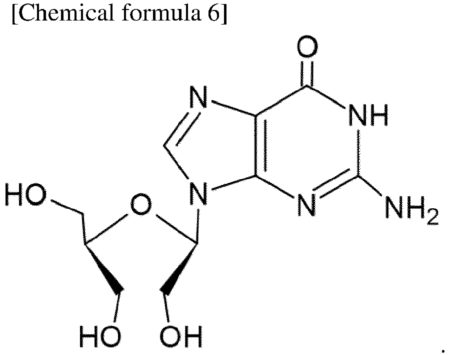

4. The composition according to claim 1 or claim 2, which is a pharmaceutical composition or food composition.

5. A feed additive comprising at least one nucleoside analogue selected from the group consisting of nucleoside analogues of the following Chemical formulas 1 to 6 and pharmaceutically acceptable salts thereof:

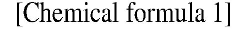

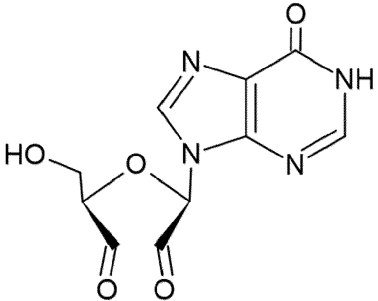

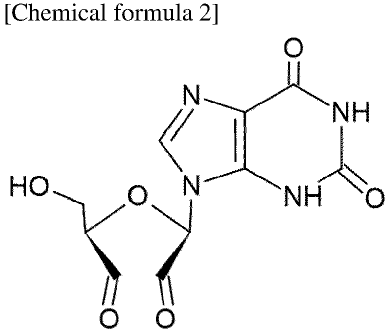

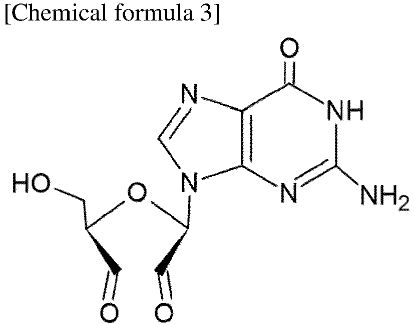

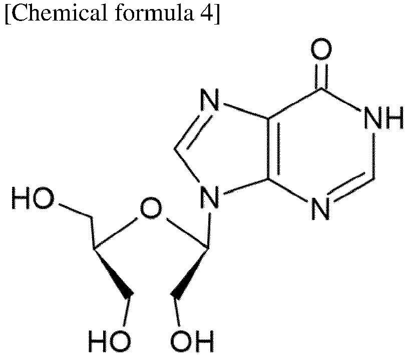

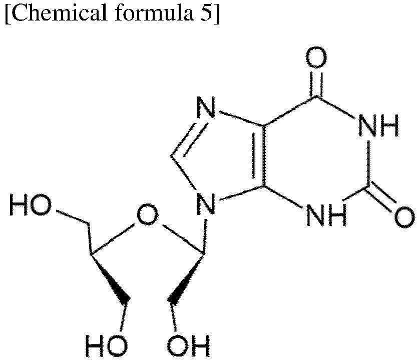

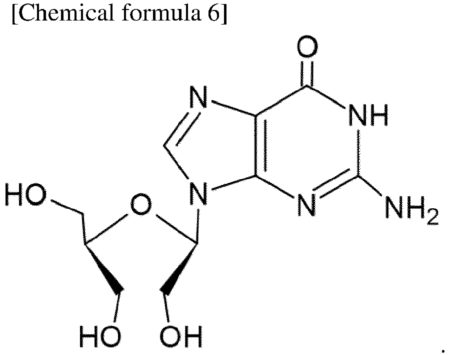

6. The feed additive according to claim 5, which is for mammals, birds, fish or arthropods.

7. A feed comprising at least one nucleoside analogue selected from the group consisting of nucleoside analogues of the following Chemical formulas 1 to 6 and pharmaceutically acceptable salts thereof:

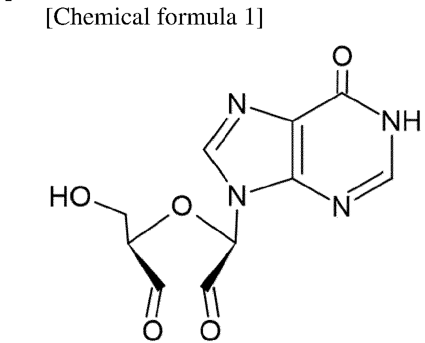

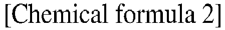

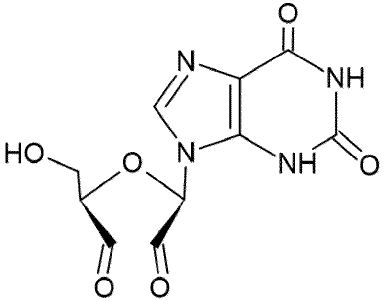

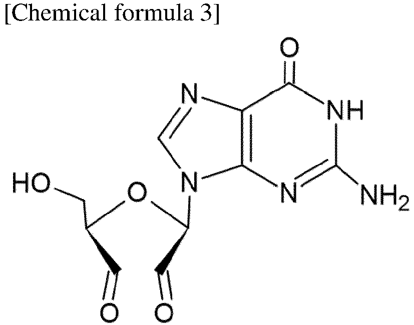

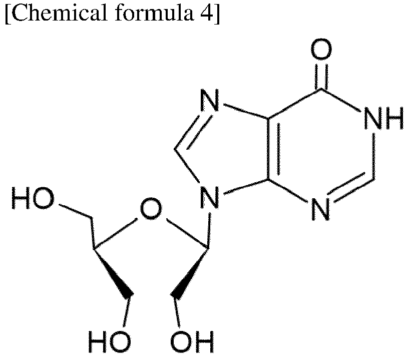

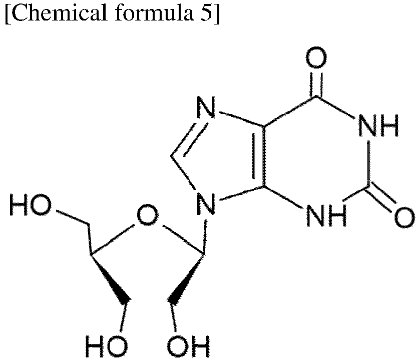

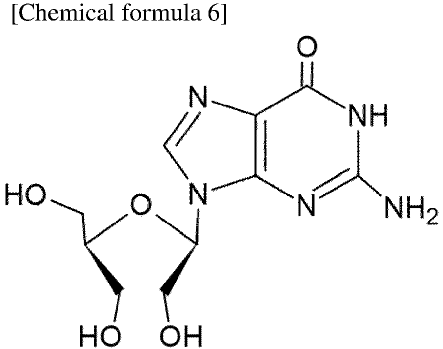

8. The feed according to claim 7, which is for mammals, birds, fish or arthropods.
